# FASCICULE DE BREVET EUROPEEN

(11) **EP 1 827 565 B1**
(45) Date de publication et mention de la délivrance du brevet: **22.07.2015**
(21) Numéro de dépôt: 05849045.9
(22) Date de dépôt: 01.12.2005
(51) Int. Cl.: A61M 37/00, A61M 5/32

(54) **DISPOSITIF POUR LA RETRO-INJECTION D'UN IMPLANT**
IMPLANTIERBARE RÜCKINJEKTIONSVORRICHTUNG
IMPLANT BACK-INJECTING DEVICE

(30) Priorité: 01.12.2004 EP 04028413; 01.12.2004 EP 04028411; 01.12.2004 EP 04028412
(43) Date de publication de la demande: 05.09.2007
(73) Titulaire: IPSEN PHARMA, 92100 Boulogne-Billancourt (FR)
(72) Inventeur: AUBERT, Christophe, 1588 Cudrefin (CH); CHERIF-CHEIKH, Roland, 08860 Castelldefels, Barcelona (ES); RIMLINGER, Thierry, 38080 L'Isle d'Abeau (FR); BONACCI, Fabrice, 69800 Saint Priest (FR); BARNEAUD, Serge, 06260 Puget Theniers (FR); SMETHAM, Grant, Timothy, Lewis, Westcott Nr Dorking, Surrey RH4 3QF (GB); DIXON, Julian, Richard, Cambridge CB4 9JJ (GB); YOUNG, Matthew, Egerton, Cambs CB4 5PZ (GB)
(74) Mandataire: Ravenel, Thierry Gérard Louis
(86) Numéro de dépôt international: PCT/EP2005/012825
(87) Numéro de publication internationale: WO 2006/058745

(56) Documents cités:
- EP-A- 0 596 162
- EP-B- 0 783 342
- US-A- 5 300 079
- US-A1- 2002 161 337
- US-B1- 6 402 716

## Description

La présente invention concerne un dispositif d'injection et, en particulier, un dispositif pour l'injection intra-musculaire ou sous-cutanée d'un principe actif pharmaceutique à l'état solide ou semi-solide habituellement appelé implant. Plus généralement, l'invention s'applique à l'injection d'un corps solide à destination humaine ou encore animale comme par exemple des puces électroniques utilisées pour l'identification d'un être vivant.

Les dispositifs de rétro-injection d'un implant du genre auquel la présente invention s'intéresse comprennent classiquement un corps principal creux auquel est fixée une aiguille creuse dans laquelle est introduit l'implant à injecter. Un corps secondaire, disposé coaxialement à l'intérieur du corps principal, entoure l'aiguille dans laquelle est susceptible de coulisser coaxialement une tige de piston, Cette tige de piston a pour but d'assurer que l'implant est déposé à la bonne profondeur dans les tissus du sujet. Lorsque l'on presse un tel dispositif de rétro-injection contre les tissus du sujet, le corps principal se met à coulisser le long du corps secondaire depuis une position proximale vers une position distale pour permettre à l'aiguille de pénétrer dans les tissus du sujet. Le déplacement du corps principal s'accompagne d'un déplacement simultané de la tige de piston qui, lors du retrait de l'aiguille hors des tissus du sujet, demeure fixe relativement à cette aiguille pour permettre de maintenir l'implant à la profondeur requise dans les tissus du sujet.

Le mode d'emploi d'un tel dispositif de rétro-injection est le suivant. En tenant d'une main le dispositif de rétro-injection par son corps principal, le praticien vient appuyer l'extrémité distale du corps secondaire contre la peau du sujet. Lorsque le dispositif de rétro-injection est convenablement disposé, le praticien exerce une poussée sur le corps principal. Sous l'effet de cette poussée, le corps principal se met à coulisser axialement le long du corps secondaire, permettant à l'aiguille qui est solidaire dudit corps principal de pénétrer dans la peau du sujet. Dans le même temps, le corps principal entraîne la tige de piston dont la position relativement à l'aiguille et à l'implant reste ainsi inchangée. C'est au moment où l'aiguille a pénétré au maximum dans les tissus du sujet que survient l'opération de rétro-injection proprement dite de l'implant. En effet, le praticien, dans un geste qui n'est pas sans rappeler celui d'une injection classique va, de sa main gauche s'il est droitier, maintenir le dispositif de rétro-injection contre la peau du sujet afin de minimiser les mouvements de l'aiguille et déplacer son autre main de manière à maintenir le corps secondaire contre la peau via son pouce qui appuie sur un bouton prévu à l'extrémité proximale du corps secondaire tandis que, de son index et de son majeur, il va commander le retour du corps principal vers sa position proximale en se servant d'un appuie-doigts dont est muni ledit corps principal. Au cours de ce geste, l'aiguille sort progressivement hors des tissus du sujet. La tige de piston n'accompagne toutefois pas ce mouvement de recul du corps principal. En effet, retenue par le corps secondaire, elle est découplée dudit corps principal et reste immobile, pénétrant ainsi progressivement dans l'aiguille creuse au fur et à mesure que cette dernière sort de la peau. L'implant émerge ainsi de l'aiguille, maintenu en position à la bonne profondeur dans la peau par l'extrémité distale de la tige de piston qui est en appui contre ledit implant. La tige de piston est alors découplée du corps secondaire et à nouveau couplée avec le corps principal de manière que l'aiguille et la tige de piston terminent ensemble le mouvement de rétro-injection dans une position dans laquelle ils sont protégés par le corps secondaire.

A l'usage, on s'est rendu compte que l'utilisation d'un dispositif de rétro-injection du genre décrit ci-dessus n'était pas toujours commode et souvent mal comprise par les praticiens. En effet, bon nombre de praticiens ont pensé que le geste était terminé une fois qu'ils avaient fait pénétrer l'aiguille au plus profond dans les tissus du sujet, omettant de procéder à l'opération de rétro-injection qui seule permet de déposer l'implant dans les tissus du sujet. D'autres praticiens ont tenté d'effectuer le geste de rétro-injection en plaçant leur pouce sur le bouton du corps secondaire et en passant leur index et leur majeur sous l'appuie-doigts comme indiqué. Toutefois, ces praticiens maintenaient fermement le corps principal de leur autre main, ce qui rendait impossible le retour dudit corps principal vers sa position proximale et donc la sortie de l'aiguille hors des tissus du sujet. Plus généralement, il a été jugé peu commode et source de douleurs pour le sujet de devoir tenir le dispositif de rétro-injection d'une main pour l'appuyer contre la peau du sujet et y enfoncer l'aiguille, puis de devoir lâcher ledit dispositif pour, de la même main, procéder au geste de rétro-injection.

La présente invention a pour but de remédier aux inconvénients susmentionnés ainsi qu'à d'autres encore en procurant un dispositif pour la rétro-injection d'un implant qui permet notamment de simplifier le plus possible le geste d'injection de l'implant sous la peau du sujet.

A cet effet, la présente invention concerne un dispositif pour la rétro-injection d'un implant dans la peau d'un sujet, ce dispositif comprenant un corps principal creux auquel est fixée une aiguille creuse dans laquelle est introduit l'implant, un corps secondaire disposé coaxialement à l'intérieur du corps principal et entourant l'aiguille, et une tige de piston susceptible de coulisser coaxialement à l'intérieur de ladite aiguille creuse et dont la position relativement à cette aiguille reste inchangée lorsque le dispositif de rétro-injection est pressé contre la peau du sujet pour permettre à l'aiguille de pénétrer dans la peau dudit sujet et que le corps secondaire se rétracte à l'intérieur du corps principal, la tige de piston s'enfonçant à l'intérieur de l'aiguille creuse pour maintenir l'implant à la profondeur requise dans la peau du sujet pendant le retrait de l'aiguille creuse hors de la peau du sujet, retrait au cours duquel le corps secondaire sort du corps principal, caractérisé en ce qu'il comprend des moyens de rappel élastiques du corps secondaire hors du corps principal.

Grâce à ces caractéristiques, la présente invention procure un dispositif pour la rétro-injection d'un implant qu'il suffit de presser contre la peau du sujet jusque tant que l'aiguille ait complètement pénétré dans la peau, le corps secondaire sortant ensuite automatiquement du corps principal pour venir recouvrir l'aiguille au fur et à mesure que l'on éloigne le dispositif de rétro-injection de la peau du sujet et que l'opération d'injection de l'implant s'effectue. L'opération d'injection de l'implant est donc entièrement automatisée sitôt l'aiguille insérée dans les tissus, ce qui la rend aussi simple que possible et permet notamment d'éliminer le geste de rétro-injection relativement compliqué auquel on devait procéder avec les dispositifs de l'art antérieur. Par ailleurs, il n'est pas nécessaire de changer la manière dont on tient le dispositif selon l'invention au cours de l'opération de rétro-injection de l'implant, ce qui facilite également le travail du praticien. On notera également que l'aiguille creuse dans laquelle est introduit l'implant est coiffée par le corps secondaire avant et après injection, ce qui évite tout risque d'éraflure et de contamination de l'aiguille et du praticien.

Selon une caractéristique complémentaire de l'invention, le dispositif de rétro-injection comprend un élément de retenue destiné à empêcher l'implant de tomber avant utilisation dudit dispositif.

Selon une première variante de réalisation, l'élément de retenue comprend une languette élastique qui, en position de repos, obture par une partie terminale inclinée vers l'intérieur du volume de l'élément de retenue le trou de passage de l'aiguille creuse et qui, lorsque le dispositif de rétro-injection est appuyé contre la peau du sujet, s'écarte pour libérer le passage de ladite aiguille creuse.

Selon une seconde variante de réalisation, l'élément de retenue comprend une languette élastique au-dessus de laquelle passe l'aiguille creuse et qui, en position de stockage du dispositif de rétro-injection, est fléchie vers l'intérieur du volume dudit élément de retenue, de sorte que l'aiguille est écartée de sa direction générale d'avance, la languette élastique recouvrant sa position de repos dans laquelle elle autorise ladite aiguille à se réaligner et à avancer lorsque l'on presse ledit dispositif de rétro-injection contre la peau du sujet.

Ces deux variantes de réalisation de l'élément de retenue de l'implant possèdent un avantage commun qui réside dans le fait que l'aiguille creuse n'a pas à venir pousser par son biseau contre un élément du dispositif d'injection pour se frayer un chemin vers la sortie dudit dispositif. Le biseau de l'aiguille ne risque donc pas de s'endommager et les risques d'injecter dans la peau du sujet des copeaux de la matière plastique dont est fait le dispositif d'injection sont évités.

Selon une autre caractéristique de l'invention, le dispositif de rétro-injection comprend en outre une gaine qui coopère avec le corps secondaire pour permettre le verrouillage irréversible du dispositif de rétro-injection après utilisation. Plus précisément, le corps secondaire se verrouille sur la gaine qui elle-même se verrouille sur le corps principal.

Le dispositif de rétro-injection est ainsi totalement verrouillé après utilisation, ce qui rend toute réutilisation ultérieure de ce dispositif impossible et surtout élimine tout risque pour le praticien de se piquer avec l'aiguille souillée.

Selon encore une autre caractéristique de l'invention, la gaine assure également le verrouillage temporaire du dispositif de rétro-injection avant utilisation.

Le dispositif de rétro-injection n'est activé qu'au moment où on le presse contre la peau du sujet, ce qui ôte toute possibilité au praticien d'accéder à l'aiguille avant de procéder à l'injection et de risquer de souiller l'aiguille en se piquant.

Selon encore une autre caractéristique de l'invention, les moyens de rappel élastiques comprennent un ressort qui est en appui à l'une de ses extrémités contre un collier lui-même en appui sur le corps secondaire, et qui est en appui à son autre extrémité contre une embase qui porte l'aiguille creuse et qui est solidaire du corps principal.

Lorsqu'on enfonce l'aiguille dans la peau du sujet, le collier remonte en coulissant axialement à l'intérieur du corps principal, de sorte que le ressort se comprime. Ensuite, arrivé à un point de rebroussement qui correspond au point de pénétration maximum de l'aiguille dans la peau, le collier pivote et coulisse à nouveau en sens inverse dans le corps principal, ce qui autorise le ressort à se détendre. Ainsi, à l'exception du collier, toutes les pièces mobiles du dispositif de rétro-injection selon l'invention ne se déplacent qu'axialement, ce qui permet de garantir un fonctionnement fiable du dispositif. En effet, lorsqu'on presse le dispositif de rétro-injection contre la peau du sujet, cela génère des forces de frottement qui pourraient gêner le bon fonctionnement du dispositif si la pièce au contact de la peau devait effectuer un mouvement de pivotement.

Selon une première variante de réalisation, le corps secondaire est un corps creux de forme sensiblement cylindrique pourvu de deux fentes rectilignes diamétralement opposées qui s'étendent depuis l'extrémité proximale dudit corps secondaire jusqu'à une hauteur déterminée au-dessus de l'extrémité distale du dispositif de rétro-injection, ces fentes matérialisant deux portions de tube.

Selon une seconde variante de réalisation, le corps secondaire comprend une seule portion de tube.

Cette seconde variante de réalisation est préférée à la première dans la mesure où elle permet de s'affranchir des problèmes de tolérance liés à la fabrication des différentes pièces constituant le dispositif de rétro-injection.

D'autres caractéristiques et avantages de la présente invention ressortiront plus clairement de la description détaillée qui suit d'un mode de réalisation du dispositif de rétro-injection selon l'invention, cet exemple étant donné à titre purement illustratif et non limitatif seulement, en liaison avec le dessin annexé sur lequel :
- la figure 1A est une vue en perspective du dispositif de rétro-injection selon l'invention en position de stockage;
- la figure 1B est une vue en coupe longitudinale du dispositif de rétro-injection représenté à la figure 1A;
- la figure 2A est une vue en perspective du dispositif de rétro-injection après retrait du capuchon;
- la figure 2B est une vue en coupe longitudinale du dispositif de rétro-injection représenté à la figure 2A;
- la figure 2C est une vue en perspective du cavalier;
- la figure 3 est une vue en coupe longitudinale du dispositif de rétro-injection appuyé contre la peau du sujet;
- la figure 4A est une vue en coupe longitudinale du dispositif de rétro-injection équipé de moyens de retenue de l'implant selon une première variante de réalisation;
- la figure 4B est une vue à plus grande échelle de la zone entourée d'un cercle sur la figure 4A;
- la figure 5A est une vue analogue à celle de la figure 4A, le dispositif de rétro-injection ayant été pressé contre la peau du sujet, les moyens de retenue s'étant écartés pour laisser le passage à l'aiguille;
- la figure 5B est une vue à plus grande échelle de la zone entourée d'un cercle sur la figure 5A;
- la figure 6A est une vue analogue à celle de la figure 5A, l'aiguille étant enfoncée dans la peau du sujet;
- la figure 6B est une vue à plus grande échelle de la zone entourée d'un cercle sur la figure 6A;
- la figure 7A est une vue partielle en perspective de la gaine, du corps secondaire et du collier;
- la figure 7B est une vue en coupe longitudinale des pièces représentées à la figure 7A;
- la figure 8A est une vue en coupe longitudinale du dispositif de rétro-injection selon l'invention en position de repos;
- la figure 8B est une vue à plus grande échelle de la zone entourée d'un cercle sur la figure 8A, la gaine étant verrouillée sur le corps principal par l'intermédiaire du corps secondaire;
- la figure 8C est une vue analogue à celle de la figure 8A, le corps secondaire ayant légèrement pénétré à l'intérieur du corps principal, découplant ainsi la gaine dudit corps principal;
- la figure 9A est une vue en coupe longitudinale du dispositif de rétro-injection équipé de moyens de retenue de l'implant selon une seconde variante de réalisation;
- la figure 9B est une vue à plus grande échelle de la zone entourée d'un cercle sur la figure 9A, les moyens de retenue étant en position de repos, empêchant l'implant de tomber;
- la figure 9C est une vue analogue à celle de la figure 9B, le dispositif de rétro-injection ayant été pressé contre la peau du sujet et les moyens de retenue autorisant l'aiguille à s'aligner avec son axe général d'avance;
- la figure 10A est une vue en perspective de l'embase servant au maintien de l'aiguille selon un premier mode de réalisation;
- la figure 10B est une vue de détail en perspective de l'extrémité proximale du corps principal;
- la figure 11A est une vue en perspective du capuchon d'extrémité;
- la figure 11B est une vue en coupe longitudinale du dispositif de rétro-injection en position de repos;
- la figure 11C est une vue à plus grande échelle de la zone entourée d'un cercle sur la figure 11A qui montre le couplage de la tête de la tige de piston sur le capuchon d'extrémité;
- la figure 12A est une vue en perspective du corps principal montrant le chemin de came ménagé dans la paroi intérieure dudit corps principal;
- la figure 12B est une vue partielle en perspective du corps principal sur laquelle sont notamment visibles les deux rainures rectilignes longitudinales du chemin de came;
- les figures 13A et 13B sont des vues en perspective face avant et face arrière du collier;
- la figure 14A est une vue en perspective du corps secondaire selon un premier mode de réalisation;
- la figure 14B est une vue de détail à plus grande échelle de la zone entourée d'un cercle sur la figure 14A;
- la figure 14C est une autre vue en perspective du corps secondaire sur laquelle sont notamment visibles les deux portions de tube;
- la figure 15 est une vue partielle en perspective montrant le corps secondaire engagé dans l'embase de l'aiguille;
- les figures 16A et 16B sont des vues schématiques montrant la coopération entre la tête de la tige de piston et les moyens de blocage prévus sur le corps secondaire;
- la figure 17A est une vue en coupe longitudinale du dispositif de rétro-injection au moment où l'aiguille a pénétré au maximum dans la peau du sujet;
- la figure 17B est une vue de détail à plus grande échelle de la zone entourée d'un cercle sur la figure 17A, la tête dé la tige de piston étant couplée avec le corps principal;
- la figure 18A est une vue en coupe longitudinale du dispositif de rétro-injection pendant la phase d'injection de l'implant dans les tissus du sujet;
- la figure 18B est une vue de détail à plus grande échelle de la zone entourée d'un cercle sur la figure 18A, la tête de la tige de piston étant découplée du corps secondaire;
- la figure 19A est une vue en coupe longitudinale du dispositif de rétro-injection après retrait complet de l'aiguille hors des tissus du sujet;
- la figure 19B est une vue à plus grande échelle de la zone entourée d'un cercle sur la figure 19A, la tête de la tige de piston étant à nouveau couplée avec le corps principal;
- les figures 20A, 20B et 20C sont des vues en coupe longitudinale du dispositif de rétro-injection respectivement avant utilisation, au moment où l'aiguille a pénétré au maximum dans la peau du sujet et après verrouillage final et qui illustrent la coopération entre le ressort et le collier;
- la figure 21A est une vue partielle en perspective du dispositif de rétro-injection montrant le collier juste avant de pivoter;
- la figure 21B est une vue analogue à celle de la figure 21A montrant le collier après pivotement;
- la figure 21C est une vue partielle en perspective montrant le collier arrivé en butée au fond des rainures les plus longues du chemin de came ménagé dans la paroi intérieure du corps principal;
- la figure 22A est une vue en coupe longitudinale du dispositif de rétro-injection après injection de l'implant dans la peau du sujet;
- la figure 22B est une vue à plus grande échelle de la zone entourée d'un cercle sur la figure 22A qui montre le corps secondaire juste avant son verrouillage sur la gaine;
- la figure 22C est une vue à plus grande échelle de la zone entourée d'un cercle sur la figure 22A qui montre le corps secondaire verrouillé sur la gaine qui elle-même est verrouillée sur le corps principal;
- les figures 23A à 23G illustrent les différentes phases de fonctionnement du dispositif de rétro-injection selon l'invention;
- la figure 24A est une vue en perspective et de dessus du corps secondaire selon un second mode de réalisation;
- la figure 24B est une vue en perspective et de dessous du corps secondaire représenté à la figure 24A;
- les figures 25A et 25B sont des vues en perspective et selon deux angles différents de l'embase de l'aiguille selon un second mode de réalisation;
- la figure 26A est une vue en coupe longitudinale avant utilisation du dispositif de rétro-injection comprenant un corps secondaire selon le second mode de réalisation;
- la figure 26B est une vue de détail à plus grande échelle de la zone entourée d'un cercle sur la figure 26A;
- la figure 27A est une vue en coupe longitudinale du dispositif de rétro-injection comprenant un corps secondaire selon le second mode de réalisation au moment où l'aiguille a pénétré au maximum dans les tissus du sujet;
- la figure 27B est une vue à plus grande échelle de la zone entourée d'un cercle sur la figure 27A juste avant que la tête de la tige de piston ne se couple avec le corps secondaire;
- la figure 27C est une vue analogue à celle de la figure 27B montrant la tête de la tige de piston couplée à la fois avec le corps principal et avec le corps secondaire;
- la figure 28A est une vue en coupe longitudinale du dispositif de rétro-injection pendant la phase de retrait de l'aiguille hors de la peau du sujet;
- la figure 28B est une vue à plus grande échelle de la zone entourée d'un cercle sur la figure 28A montrant la tête de la tige de piston solidaire du corps secondaire et glissant par son ergot contre la paroi intérieure du corps principal;
- la figure 29A est une vue en coupe longitudinale du dispositif de rétro-injection sur laquelle l'aiguille est pratiquement retirée de la peau du sujet;
- les figures 29B et 29C sont des vues à plus grande échelle de la zone entourée d'un cercle sur la figure 29A qui montre comment la tête de la tige de piston se couple à nouveau avec le corps principal;
- la figure 30 est une vue en coupe longitudinale du dispositif de rétro-injection en position verrouillée après injection de l'implant, la tête de la tige de piston étant couplée avec le corps principal;
- la figure 31 est une vue partielle en perspective et en coupe de l'extrémité arrière du dispositif de rétro-injection montrant un autre moyen de couplage de la tête de la tige de piston avec le corps secondaire, et
- la figure 32 est une vue en perspective montrant la coopération entre le corps secondaire et l'embase selon le second mode de réalisation.

Dans tout ce qui suit, on entendra par extrémité "proximale" l'extrémité située du côté du praticien, et par extrémité "distale" l'extrémité située du côté du sujet auquel l'injection est administrée.

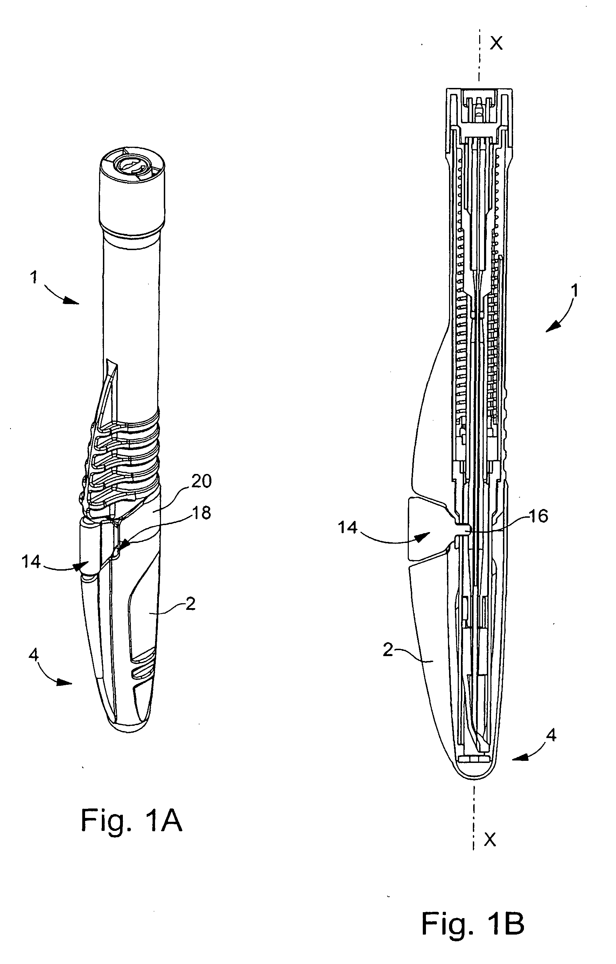

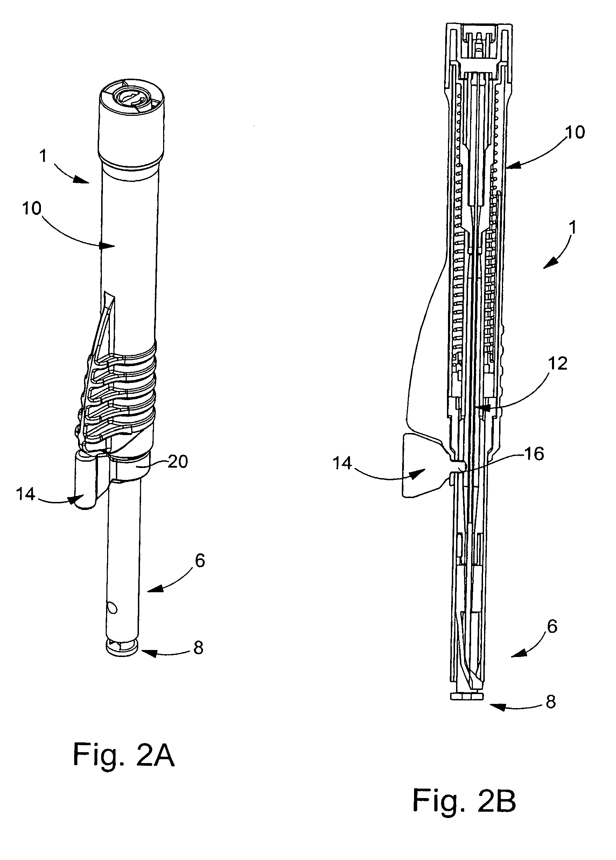

On commence par sortir le dispositif de rétro-injection selon l'invention de son emballage secondaire (non représenté). Désigné dans son ensemble par la référence numérique générale 1, le dispositif de rétro-injection comprend en premier lieu (voir figures 1A et 1B) un capuchon 2 qui coiffe son extrémité distale 4. Pour pouvoir procéder à la rétro-injection, il faut tout d'abord retirer ce capuchon 2 en exerçant sur lui une légère force de traction selon l'axe longitudinal X-X dudit dispositif de rétro-injection 1. Au cours de ce mouvement, on découvre (voir figures 2A et 2B) une gaine 6 prolongée à son extrémité distale par un élément de retenue 8, ces deux éléments faisant saillie du corps principal 10 du dispositif de rétro-injection 1. Comme on le verra plus en détail ci-après, l'élément de retenue 8 est solidaire d'un corps secondaire 12 qui s'étend coaxialement à l'intérieur du corps principal 10.

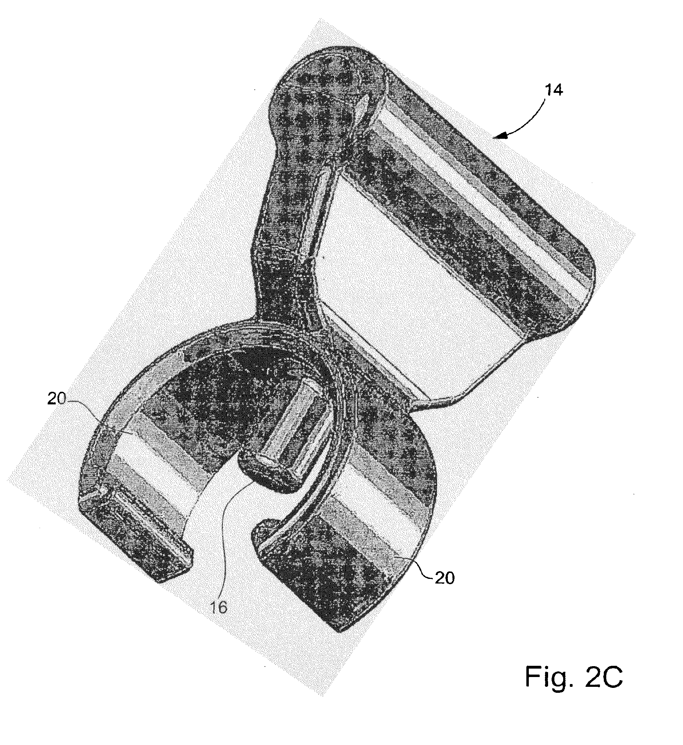

L'opération de rétro-injection ne peut débuter avant que l'on ait en outre retiré un cavalier 14 qui présente (voir figure 2C) à sa base un téton 16 par lequel il est engagé dans des trous correspondants pratiqués respectivement dans le corps principal 10, la gaine 6 et le corps secondaire 12. Ainsi, aussi longtemps que le cavalier 14 est en place, le corps principal 10, la gaine 6 et le corps secondaire 12 sont couplés les uns avec les autres, ce qui rend tout déplacement axial de ces éléments les uns par rapport aux autres impossible et empêche donc de procéder à la rétro-injection. On notera que le capuchon 2 présente une échancrure 18 par laquelle il vient recouvrir les bras 20 du cavalier 14 qui épousent la forme sensiblement circulaire de l'extrémité distale du corps principal 10. Il est ainsi impossible de retirer le cavalier 14 avant d'avoir ôté le capuchon 2, ce qui offre une sécurité supplémentaire contre toute activation involontaire du dispositif de rétro-injection 1 selon l'invention.

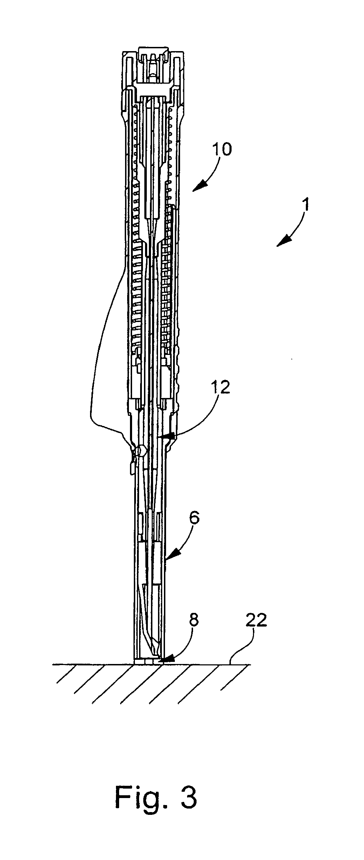

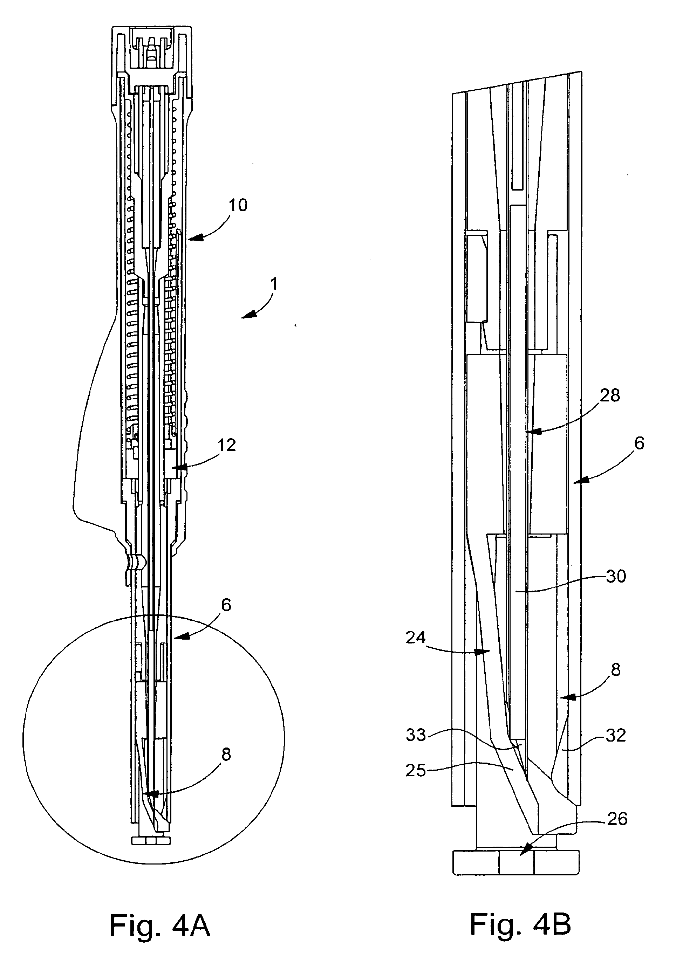

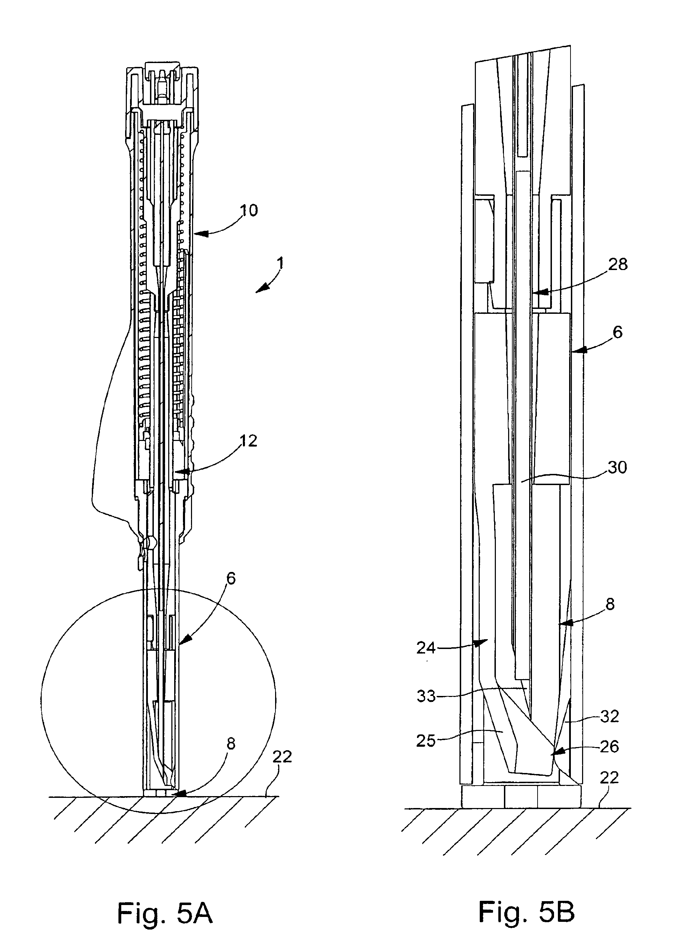

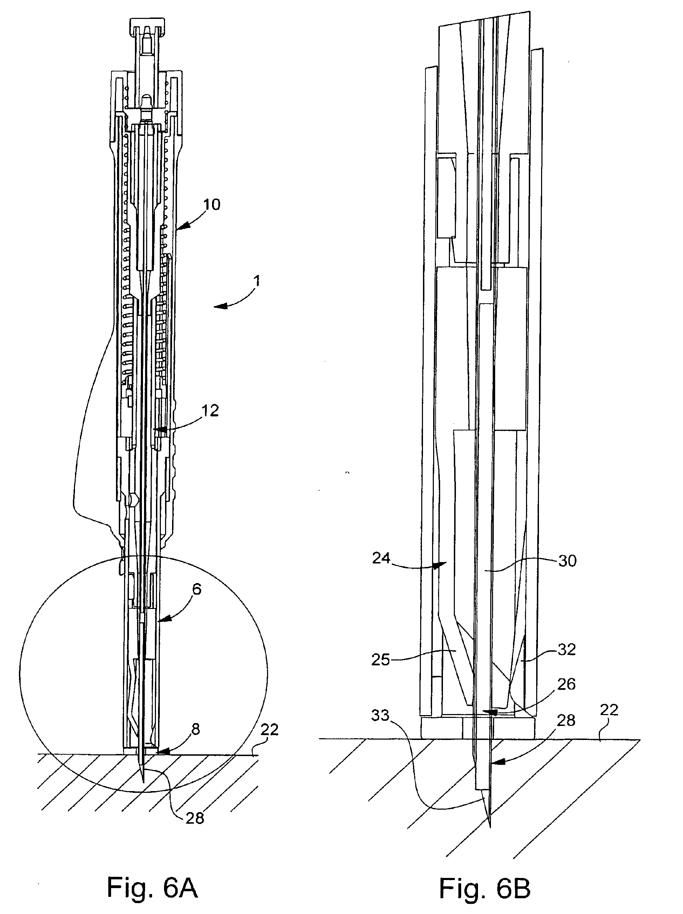

Après retrait du capuchon 2 et du cavalier 14, l'opération de rétro-injection proprement dite peut débuter. Pour cela, le praticien active le dispositif d'injection 1 en l'appuyant par son élément de retenue 8 contre la peau du sujet 22. Sous l'effet de cette poussée, l'élément de retenue 8 pénètre légèrement à l'intérieur de la gaine 6 (voir figure 3). Or, cet élément de retenue 8 comprend (voir figures 4A et 4B) une languette élastique 24 qui, en position de repos, obture par une partie terminale 25 inclinée vers l'intérieur du volume de l'élément de retenue 8 le trou de passage 26 d'une aiguille creuse 28 disposée coaxialement à l'intérieur du corps secondaire 12 et dans laquelle est engagé l'implant 30 destiné à être administré au sujet. Comme la gaine 6 présente sur le pourtour intérieur de son extrémité distale deux plans inclinés 32 qui vont venir appuyer sur la languette élastique 24 lors du mouvement d'avance de l'élément de retenue 8 à l'intérieur de ladite gaine 6 (voir figures 5A et 5B), ladite languette 24 va s'écarter de sa position de repos et ainsi libérer le passage de l'aiguille 28 (voir figures 6A et 6B). L'aiguille creuse 28 n'est ainsi pas obligée de se frayer un chemin en poussant pas son biseau 33 un élément du dispositif 1, ce qui permet de ne pas endommager ledit biseau 33 et d'éviter de risquer d'injecter des copeaux de matière plastique dans la peau 22 du sujet. On notera également que les plans inclinés 32 servent à indexer la position de l'élément de retenue 8 à l'intérieur de la gaine 6. A cet effet, l'élément de retenue 8 présente une nervure légèrement en saillie par rapport à sa surface cylindrique extérieure et qui vient glisser entre lesdits deux plans inclinés 32.

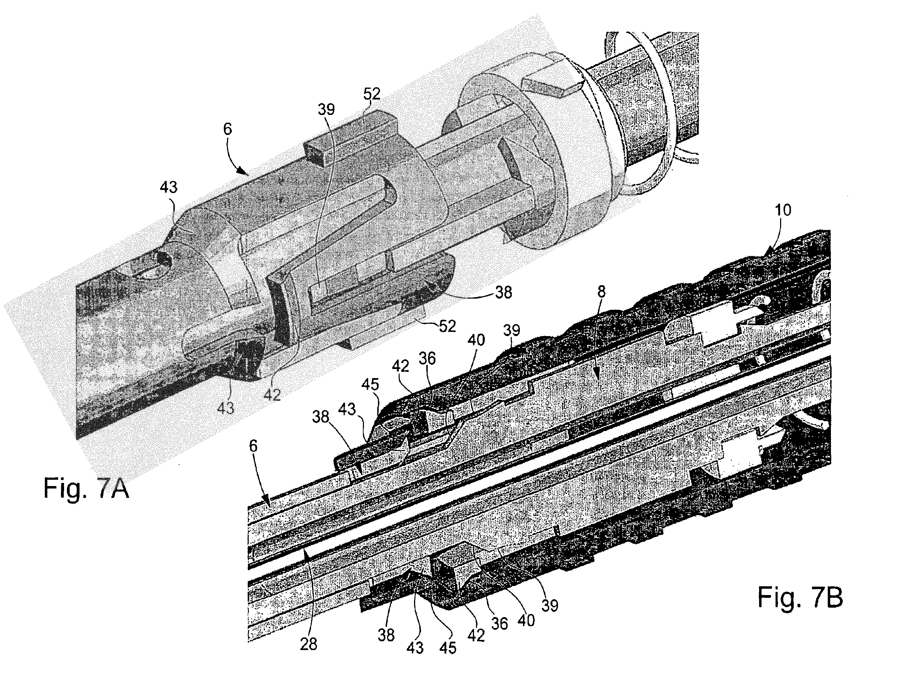

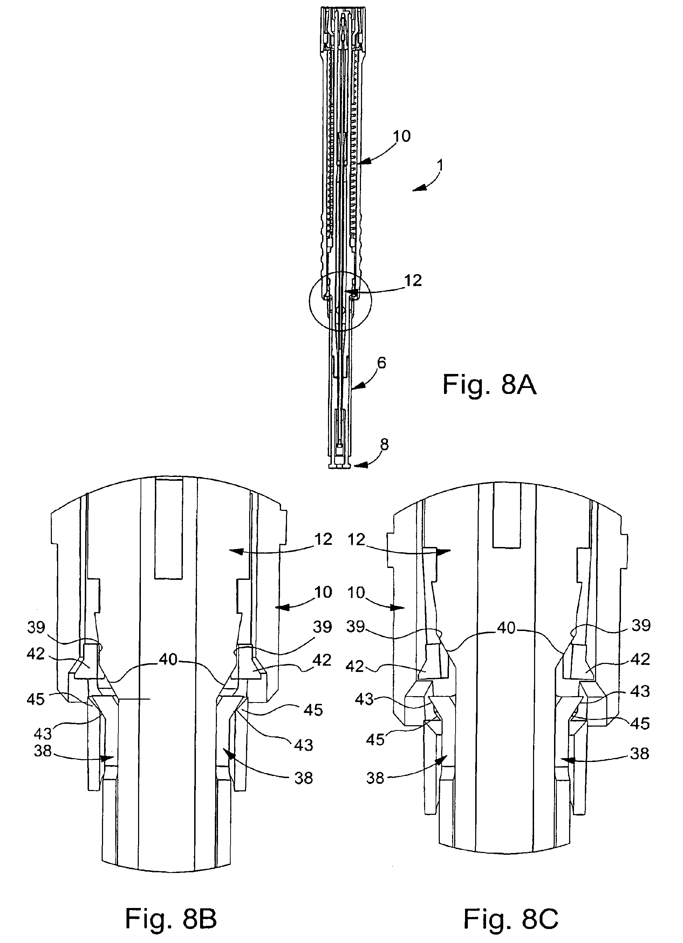

Le corps principal 10 présente sur le pourtour intérieur de son extrémité distale au moins un et, préférentiellement, deux logements 36 diamétralement opposés et dans lesquels viennent faire saillie deux bras élastiques 38 sensiblement en forme de V prévus à l'extrémité proximale de la gaine 6 (voir figures 7A et 7B). Ces deux bras 38 ont deux fonctions. La première de ces fonctions est de permettre l'immobilisation temporaire de ladite gaine 6 relativement audit corps principal 10 en position de stockage du dispositif de rétro-injection 1 selon l'invention. A cet effet, le corps secondaire 12 présente sur son pourtour extérieur deux portions planes 39 diamétralement opposées et qui, en position de stockage, maintiennent les bras élastiques 38 de'la gaine 6 dans des logements 36 ménagés dans la surface intérieure du corps principal 10 (voir figure 8B). On notera que les bras élastiques 38 présentent chacun à leur base un ergot 42 qui vient faire saillie dans le logement correspondant 36. Par suite, lorsqu'on appuie le dispositif de rétro-injection 1 par l'extrémité distale de son élément de retenue 8 contre la peau 22 du sujet, ledit élément de retenue 8, solidaire du corps secondaire 12, fait légèrement remonter ce dernier à l'intérieur du corps principal 10, de sorte que des plans inclinés 40 ménagés sur le pourtour extérieur dudit corps secondaire 12 s'éloignent des bras élastiques 38 qui sont ainsi libres de fléchir (voir figure 8C). Les ergots 42 se dégagent alors de leur position en prise dans les logements 36, de sorte que la gaine 6 se retrouve découplée du corps principal 10 et est autorisée à remonter à l'intérieur de celui-ci. On notera par ailleurs que la gaine 6 présente deux rebords tronconiques 43 par lesquels elle vient en butée contre un épaulement intérieur 45 prévu au niveau de l'extrémité distale du corps principal 10.

La seconde fonction des bras élastiques 38 est de permettre le verrouillage final du corps secondaire 12 sur la gaine 6 comme il sera décrit ultérieurement.

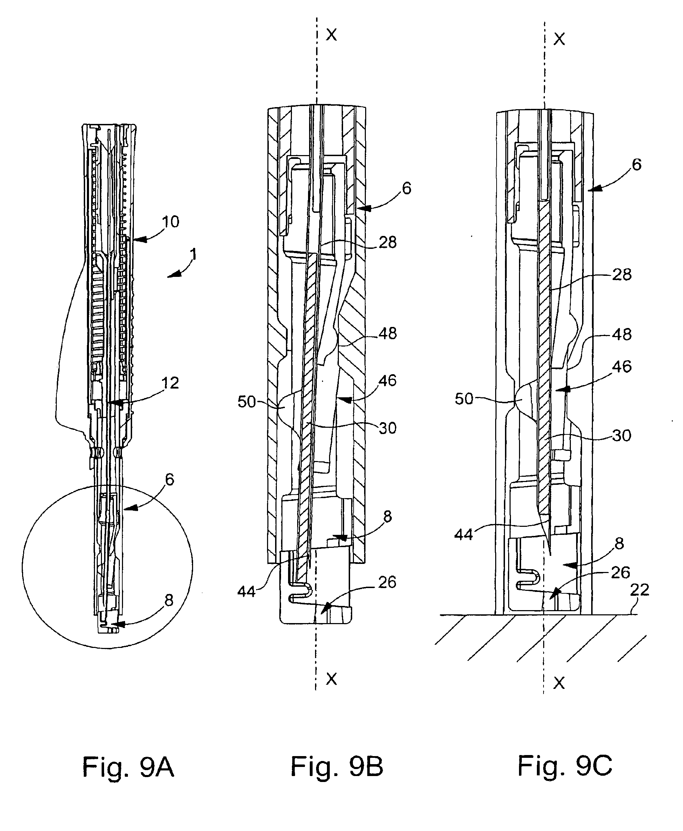

Comme on l'a vu ci-dessus, une première solution envisageable pour retenir l'aiguille creuse 28 et prévenir la chute de l'implant 30 avant injection est de prévoir sur le chemin de ladite aiguille 28 une languette élastique 24 qui, en position de repos, obture le biseau 33 de l'aiguille 28 et qui, en position activée, est écartée du chemin de cette aiguille 28 pour permettre l'avance de cette dernière et le dépôt de l'implant 30 dans les tissus 22 du sujet. Selon une variante préférée (voir figures 9A et 9B), l'élément de retenue 8 comprend une languette élastique 46 au-dessus de laquelle passe l'aiguille 28 et qui, dans la position de stockage du dispositif de rétro-injection 1, est fléchie vers l'intérieur du volume de l'élément de retenue 8 par un ergot 48 prévu sur la surface intérieure de la gaine 6. L'aiguille 28 est ainsi légèrement écartée de sa direction générale d'avance suivant l'axe de symétrie longitudinale X-X du dispositif de rétro-injection 1 de sorte que, si l'implant glisse partiellement hors de l'aiguille 28 sous l'effet de son poids, il viendra buter contre l'extrémité distale de l'élément de retenue 8, ce qui l'empêchera de tomber. Par suite, lorsqu'on appuie le dispositif de rétro-injection 1 contre la peau 22 du sujet, l'élément de retenue 8 pénètre légèrement à l'intérieur de la gaine 6, de sorte que l'ergot 48 s'éloigne de la languette 46 qui recouvre sa position de repos et qui autorise l'aiguille 28 à s'aligner avec l'axe de symétrie longitudinale X-X du dispositif d'injection 1 et le trou de passage 26. Additionnellement, une arche 50 peut être prévue en extrémité de la languette 46 pour un meilleur guidage axial de l'aiguille creuse 28.

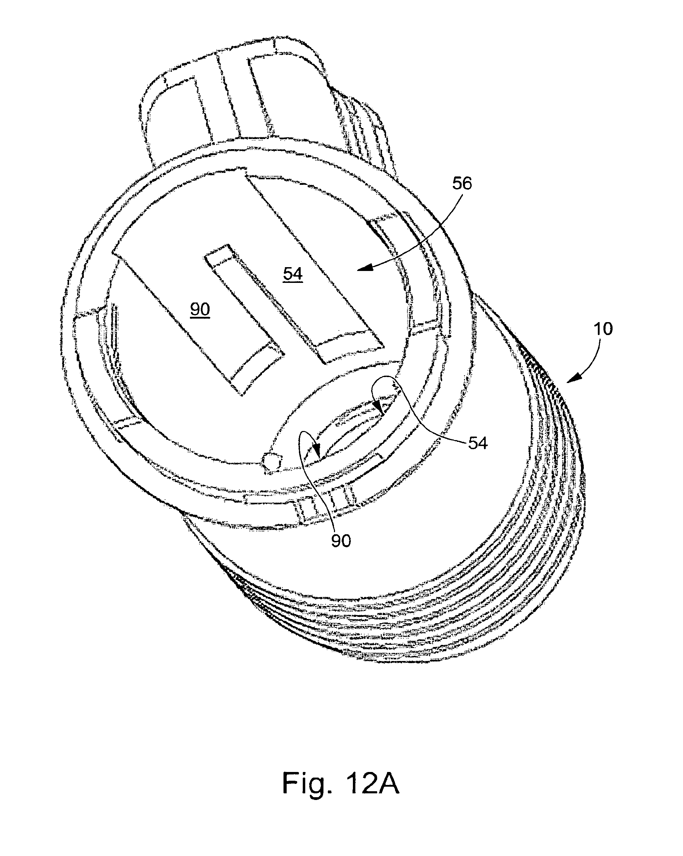

La position du corps secondaire 12 est indexée par rapport à la gaine 6 par le biais de ses deux plans inclinés 40 qui doivent être glissés dans les creux des bras élastiques 38 en forme de V. Quant à la position de la gaine 6 relativement au corps principal 10, elle est indexée par au moins un et, préférentiellement, deux ergots 52 (voir figure 7A) diamétralement opposés qui sont engagés dans les rainures les plus longues 54 de deux chemins de came 56 ménagés dans la paroi latérale intérieure du corps principal 10 (voir figure 12A).

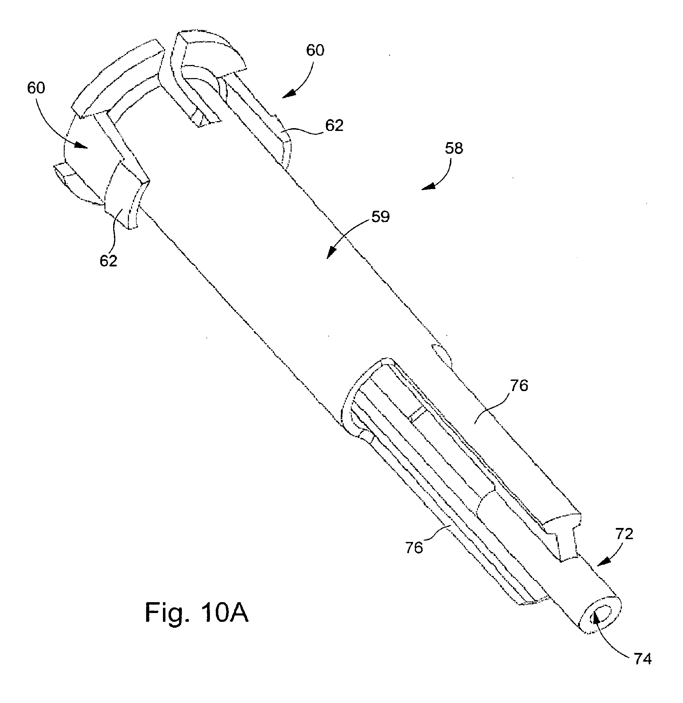

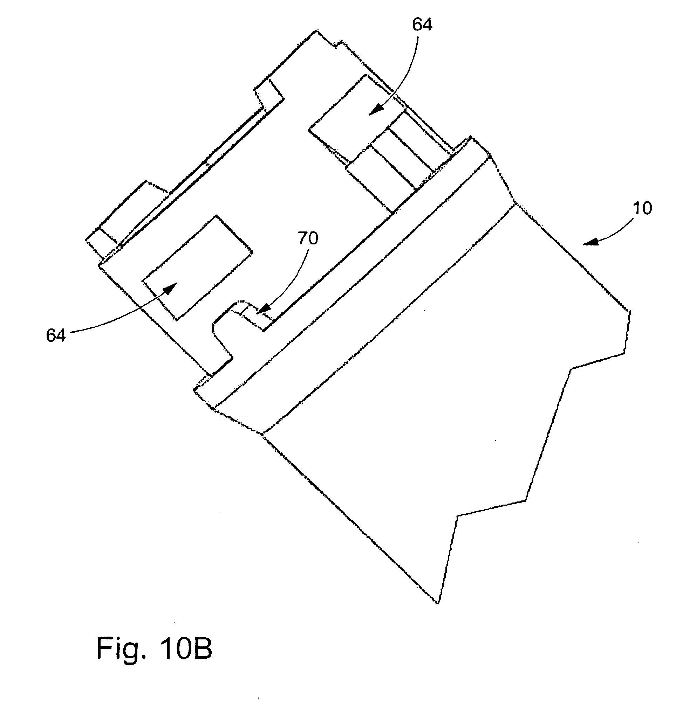

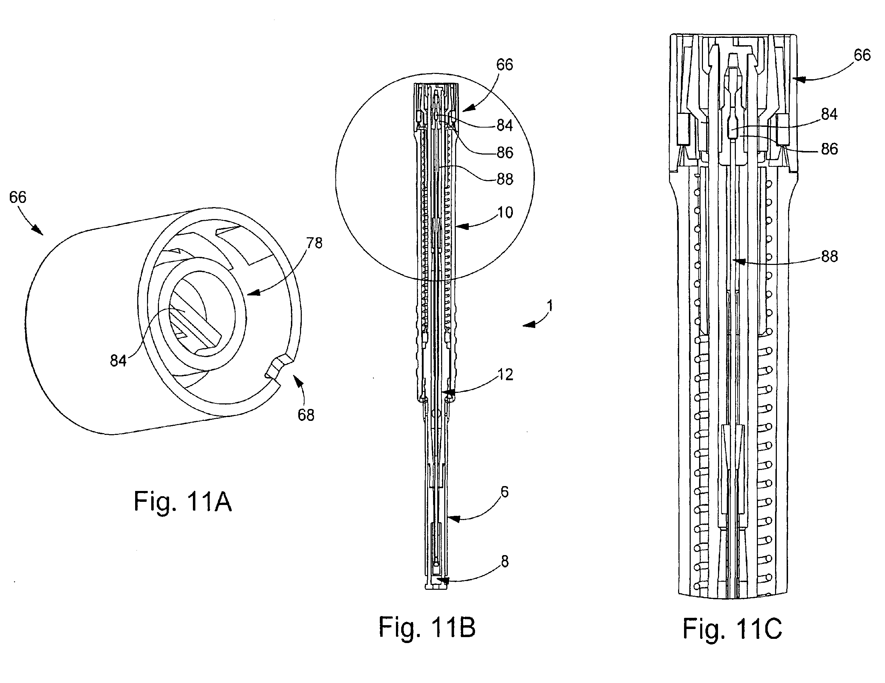

L'aiguille creuse 28 est portée (voir figure 10A) par une pièce de maintien sensiblement cylindrique appelée embase et désignée dans son ensemble par la référence numérique générale 58. L'extrémité proximale de l'embase 58 est découpée en créneaux qui forment des languettes 60 pourvues à leur base de renflements 62. Ces renflements 62 font saillie dans des ouvertures correspondantes 64 ménagées au niveau de l'extrémité proximale du corps principal 10 pour le verrouillage de l'embase 58 sur ledit corps principal 10 (voir figure 10B). Par ailleurs, l'embase 58 est coiffée par un capuchon d'extrémité 66 (voir figure 11A) qui présente à sa base une encoche 68 dans laquelle vient se loger un index 70 prévu sur le corps principal 10.

Vers son extrémité distale, l'embase 58 comprend une portion de tube 72 qui définit une ouverture traversante 74 pour le maintien à friction et/ou collage de l'aiguille creuse 28 et qui est reliée au corps cylindrique creux 59 de l'embase 58 par une et, préférentiellement, deux nervures 76 diamétralement opposées.

Une douille 78 de forme cylindrique s'étend coaxialement à l'intérieur du capuchon d'extrémité 66. Comme décrit en détail ci-dessous, le capuchon 66 comprend également une barre 84 qui s'étend selon un diamètre à l'intérieur de la douille 78 et sur laquelle vient se fixer par pincement la tête 86 d'une tige de piston 88 apte à coulisser à l'intérieur de l'aiguille creuse 28 pour maintenir en place l'implant 30 au moment de la rétro-injection (voir figure 11C).

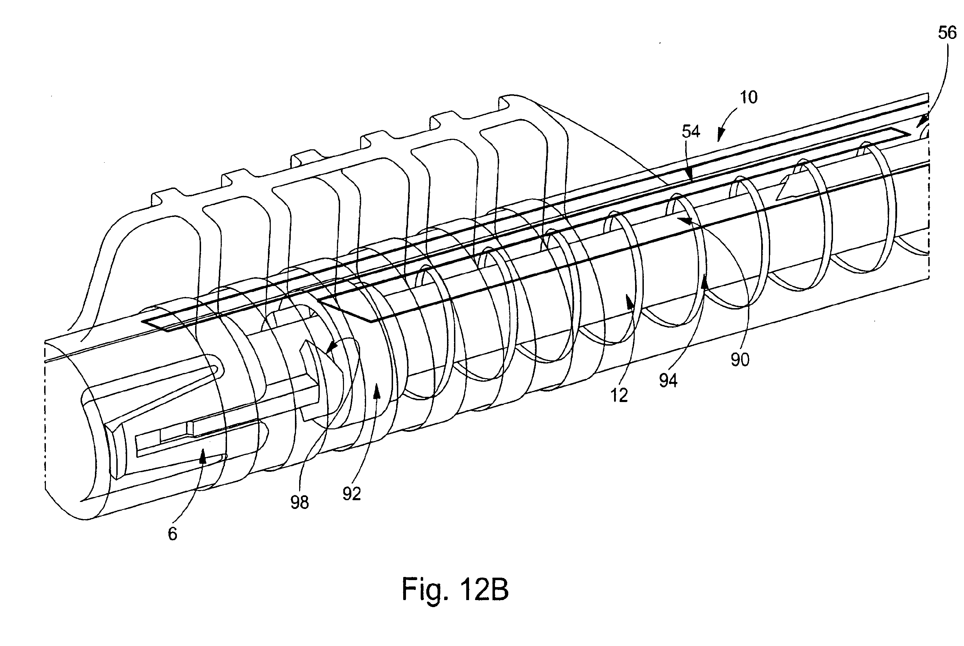

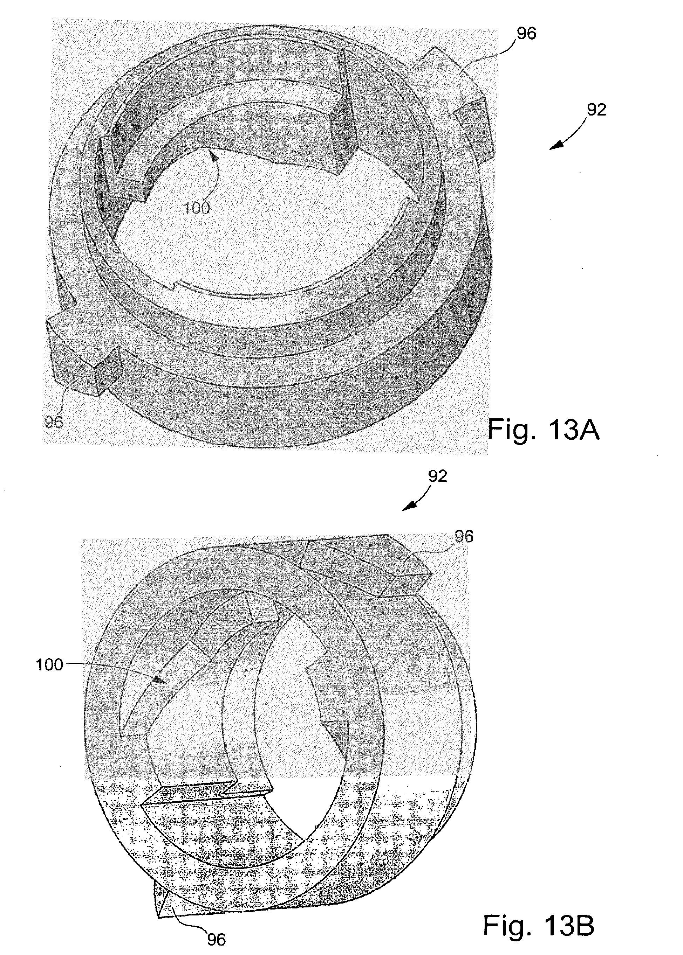

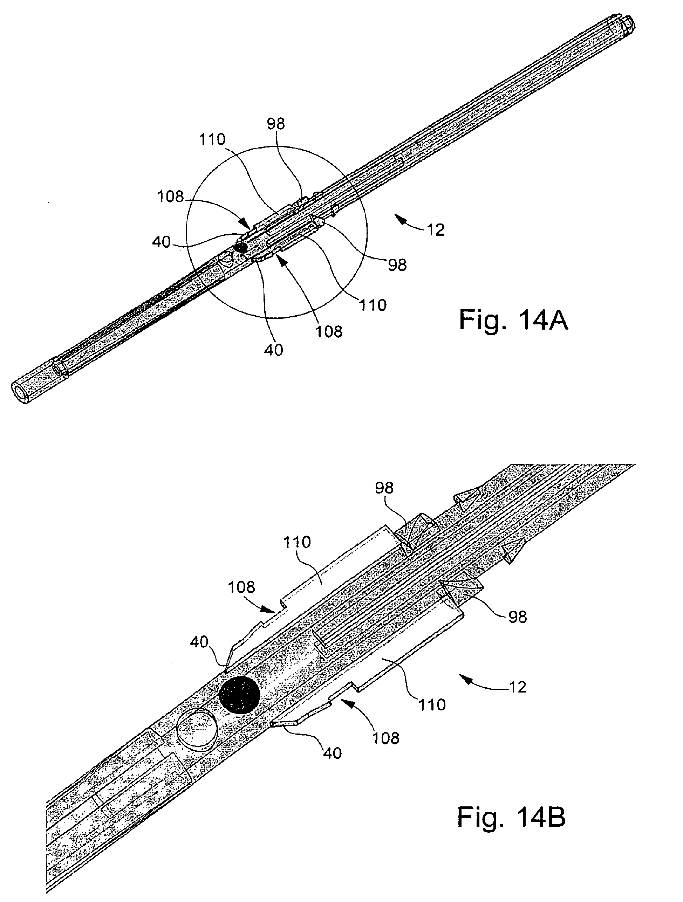

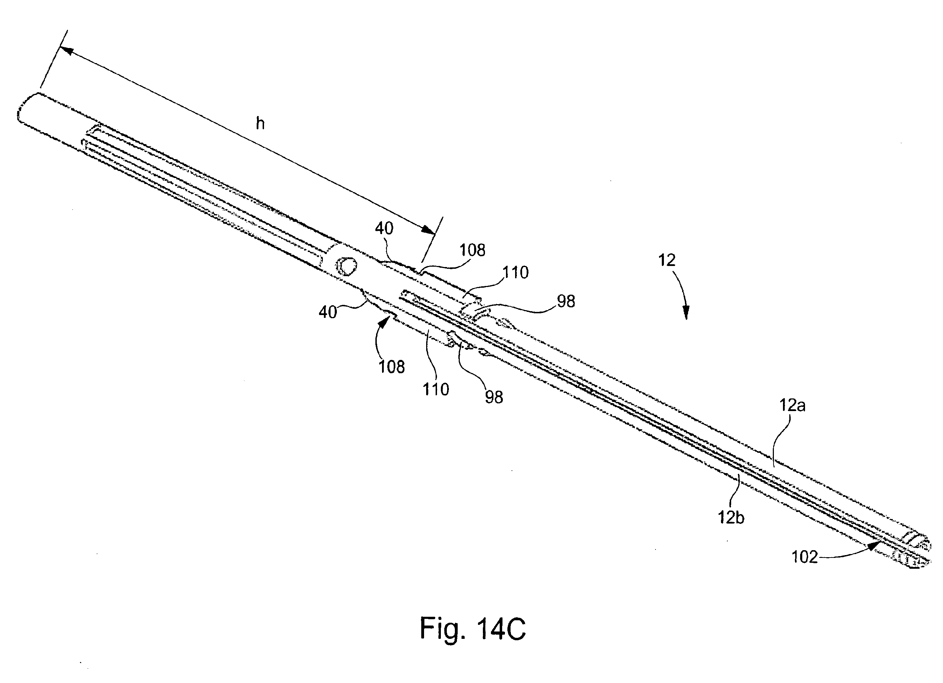

Comme on l'a déjà mentionné ci-avant, le corps principal 10 présente sur sa surface intérieure au moins un et, préférentiellement, deux chemins de came 56 diamétralement opposés et formés chacun de deux rainures rectilignes longitudinales 54 et 90 dont l'une 54, est plus longue que l'autre 90. Un collier 92 sert notamment de pièce d'appui à un ressort 94 (voir figures 12A et 12B). En position de stockage du dispositif de rétro-injection 1, ce ressort 94 est légèrement précontraint. Comme on le verra ci-après, ce ressort 94 va se comprimer davantage dès que l'on commence à appuyer ledit dispositif de rétro-injection 1 contre la peau 22 du sujet et sera à nouveau autorisé à se détendre au moment de la rétro-injection proprement dite. Le collier 92 est apte à coulisser à l'intérieur du corps principal 10 et présente à cet effet à sa périphérie deux ergots 96 diamétralement opposés qui sont susceptibles de coopérer chacun avec l'une ou l'autre des deux rainures 54, 90 des chemins de came 56 en fonction de la position angulaire dudit collier 92 à l'intérieur dudit corps principal 10 (voir figures 13A et 13B). Cette position est contrôlée par le corps secondaire 12 qui présente à cet effet une surface de came 98 (voir figures 14A à 14C) contre laquelle le collier 92 vient en appui par un chemin de came 100 ménagé sur sa surface intérieure. Lorsque les ergots 96 portés par le collier 92 sortent des rainures 90 les plus courtes, ledit collier 92, contraint par le ressort 94, glisse par son chemin de came 100 le long de la surface de came 98 du corps secondaire 12, ce qui l'oblige à pivoter, ses ergots 96 pénétrant alors dans les rainures 54 les plus longues, autorisant le ressort 94 à se détendre à nouveau.

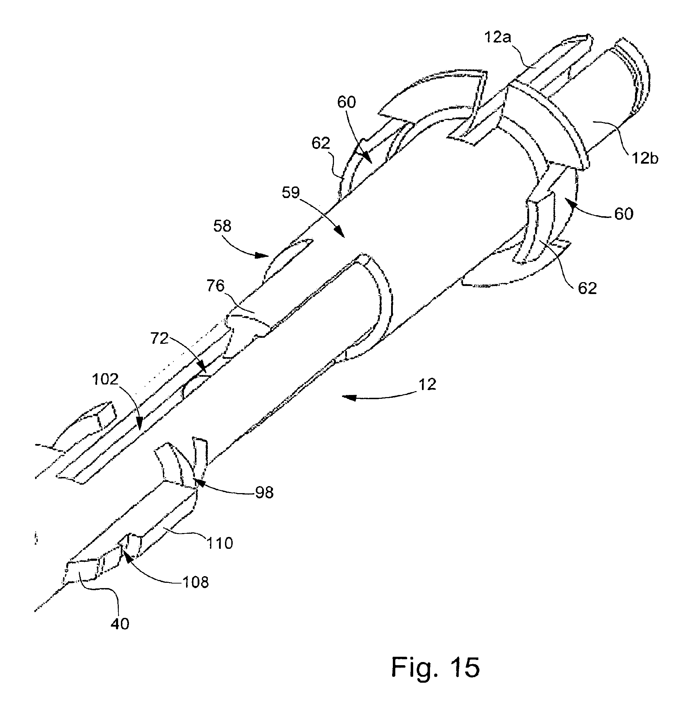

Le corps secondaire 12 est un corps creux de forme généralement cylindrique pourvu de deux fentes rectilignes 102 diamétralement opposées et qui s'étendent depuis l'extrémité proximale dudit corps secondaire 12 jusqu'à une hauteur h au-dessus de l'extrémité distale 4 du dispositif de rétro-injection 1. Comme on va le voir ci-après, cette hauteur h détermine la profondeur de pénétration de l'aiguille creuse 28 dans la peau 22 du sujet. En effet, les fentes 102 matérialisent deux portions de tube 12a et 12b qui passent de part et d'autre de la portion de tube 72 dans laquelle est fixée l'aiguille creuse 28 et qui pénètrent à l'intérieur du corps cylindrique creux 59 de l'embase 58 (voir figure 15). Ainsi, cette embase 58 est libre de glisser le long du corps secondaire 12 jusqu'à tant que les nervures 76 par lesquelles la portion de tube 72 est reliée au corps 59 de ladite embase 58 arrivent en butée au fond des fentes 102. Par ailleurs, les deux portions de tube 12a, 12b passent de part et d'autre de la barre 84 et pénètrent à l'intérieur de la douille 78 dont elles ressortent par l'extrémité proximale 103 du dispositif de rétro-injection 1. Les deux portions de tube 12a, 12b du corps secondaire 12 sont donc capables d'émerger progressivement du corps principal 10 au fur et à mesure que l'aiguille creuse 28 pénètre dans la peau 22 du sujet.

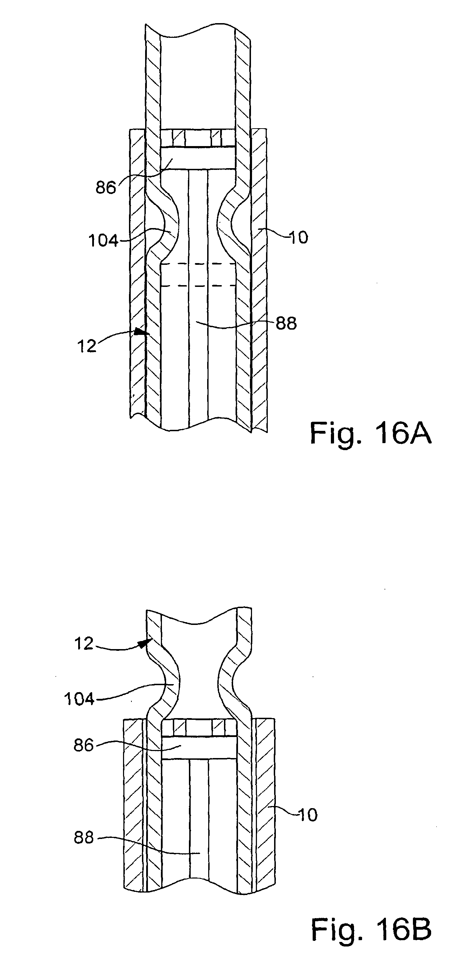

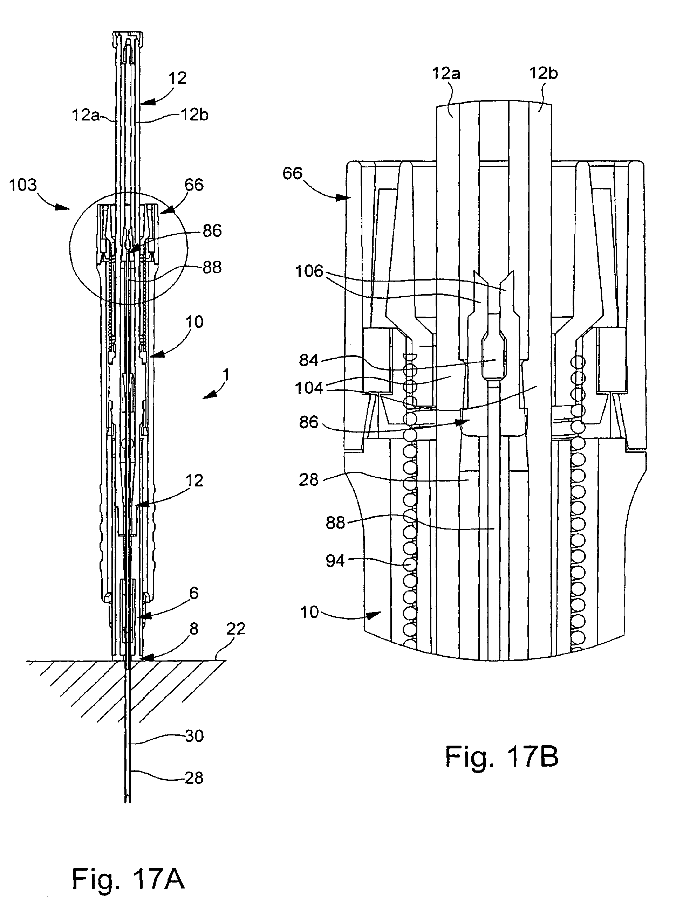

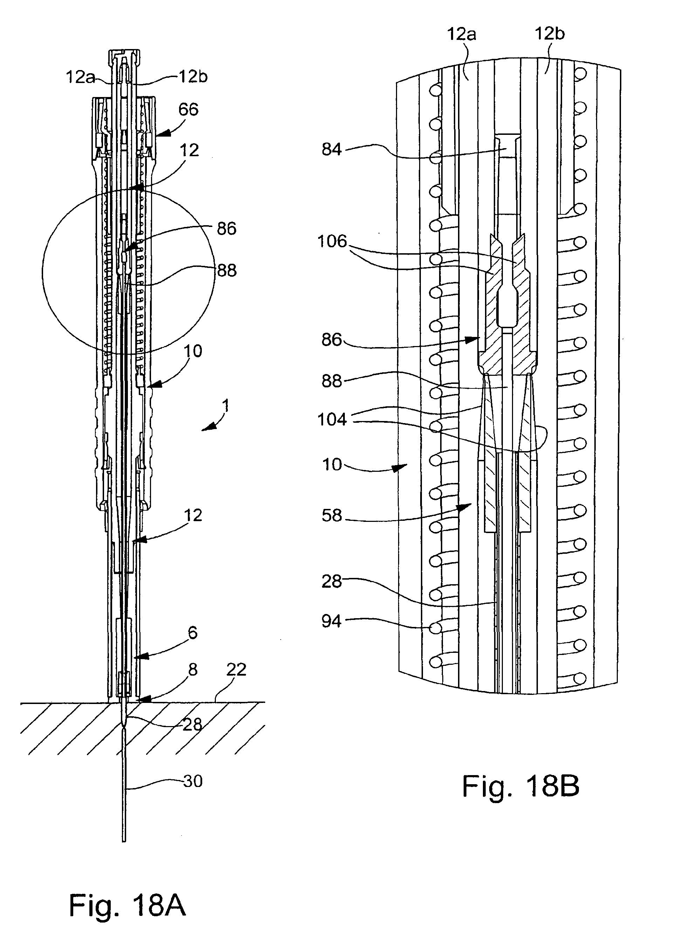

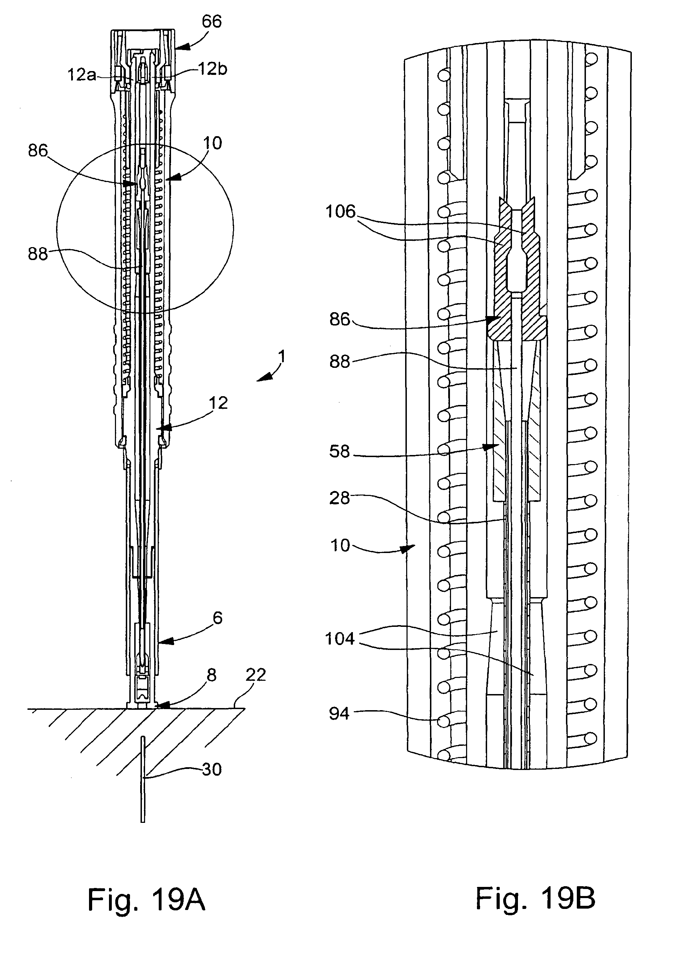

Enfin, le corps secondaire 12 comprend sur la face intérieure des deux portions de tube 12a, 12b des moyens de blocage 104, par exemple sous la forme de deux bourrelets (voir figure 16A et 16B). Pour une description détaillée de ces moyens de blocage 104, on se réfèrera utilement à la demande de brevet européen No. 04028413.5 au nom de la Demanderesse. Les moyens de blocage 104 ont pour fonction de découpler la tête 86 de la tige de piston 88 du corps principal 10 et de la coupler avec le corps secondaire 12. En effet, avant utilisation, la tête 86 de la tige de piston 88 est pincée par le biais de deux clips élastiques 106 sur la barre 84 qui s'étend diamétralement à l'intérieur de la douille 78 du capuchon d'extrémité 66. Au fur et à mesure que l'aiguille creuse 28 pénètre dans la peau 22 du sujet, les deux portions de tube 12a, 12b du corps secondaire 12 émergent progressivement du corps principal 10, de sorte que les deux bourrelets 104 viennent à la rencontre de la tête 86 de la tige de piston 88 et finissent par passer derrière celle-ci en se déformant élastiquement (voir figures 17A et 17B). Lors de l'opération de rétro-injection proprement dite, on commande le retrait progressif de l'aiguille creuse 28 hors de la peau 22 du sujet. La tige de piston 88 n'accompagne toutefois pas ce mouvement de recul (voir figures 18A et 18B). En effet, sa tête 86 étant retenue par les bourrelets 104, elle est découplée du corps principal 10 (plus précisément du capuchon d'extrémité 66) et reste immobile, pénétrant ainsi progressivement dans l'aiguille creuse 28 au fur et à mesure que cette dernière sort de la peau 22 du sujet. L'implant 30 émerge ainsi de l'aiguille 28, maintenu en position à la bonne profondeur dans la peau 22 par l'extrémité distale de la tige de piston 88 qui est en appui contre ledit implant 30. Au cours du mouvement de descente du corps secondaire 12 relativement au corps principal 10, l'extrémité proximale de l'embase 58 vient à la rencontre de la tête 86 de la tige de piston 88. Comme lors du mouvement de montée du corps secondaire 12 à l'intérieur du corps principal 10, les moyens de blocage 104 que porte le corps secondaire 12 vont dépasser la tête 86 de la tige de piston 88 en se déformant élastiquement, découplant ainsi la tête 86 de la tige de piston 88 du corps secondaire 12 et la couplant à nouveau avec le corps principal 10 pour autoriser la poursuite du mouvement de descente du corps secondaire 12 et lui permettre ainsi de recouvrir l'aiguille 28 et la tige de piston 88 (voir figures 19A et 19B).

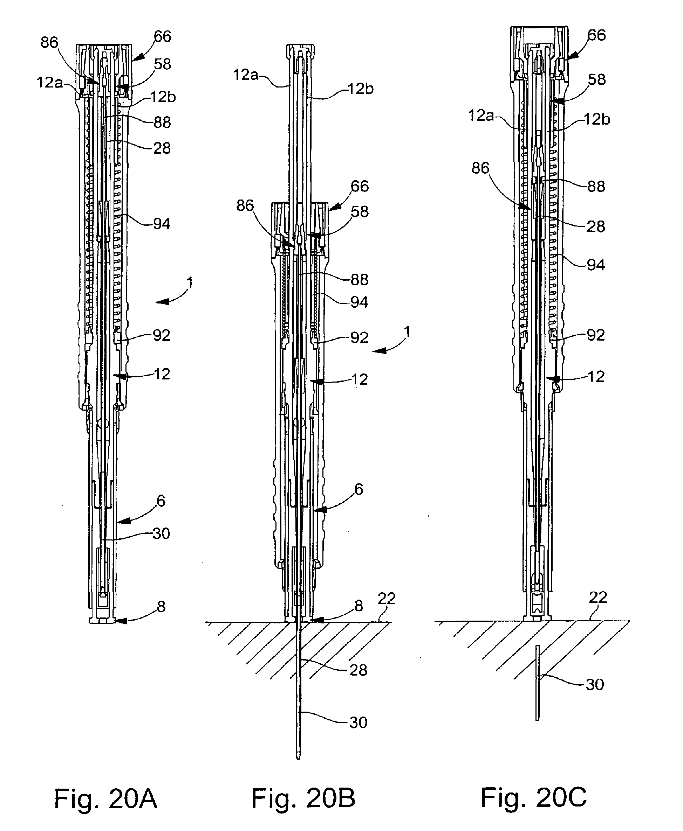

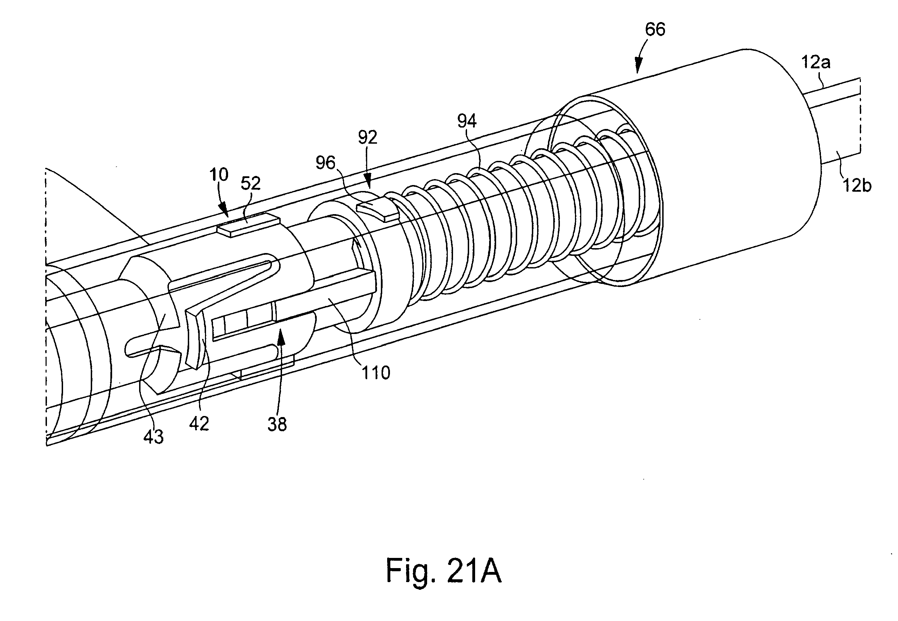

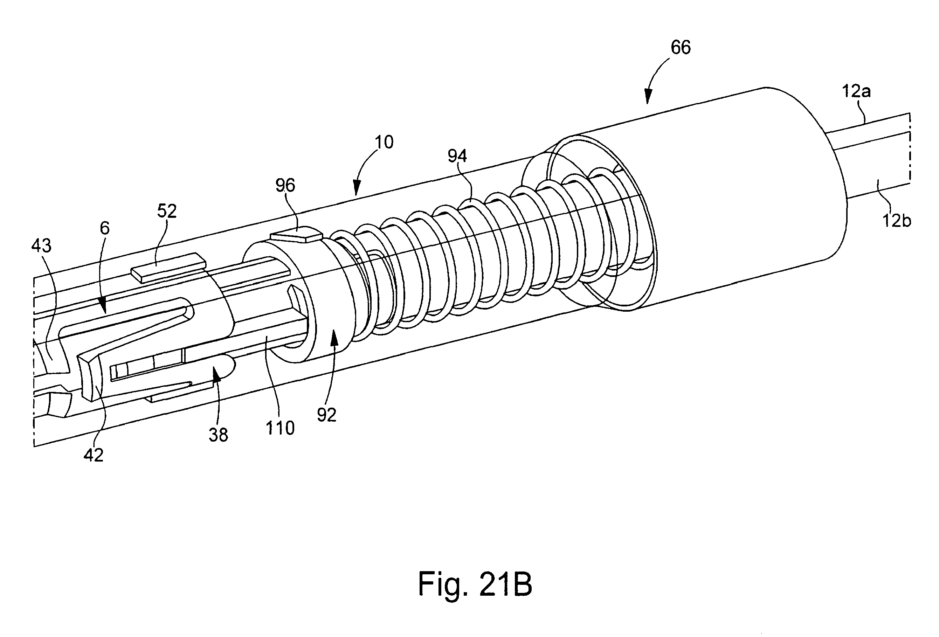

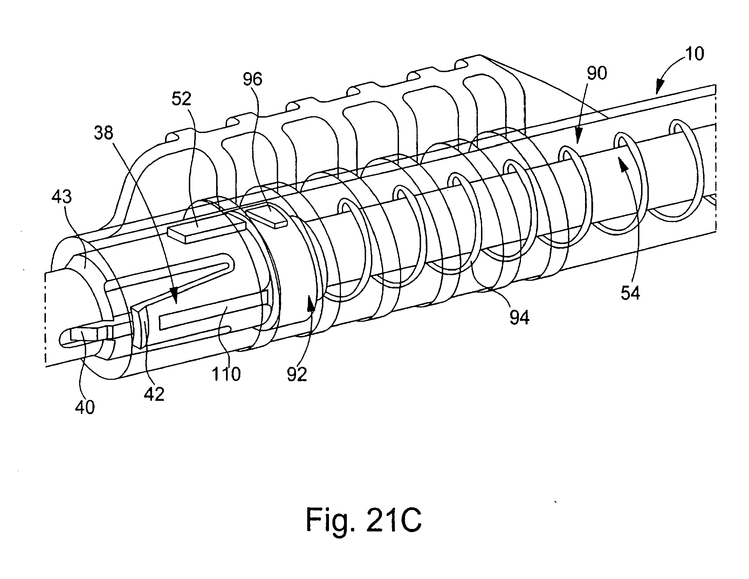

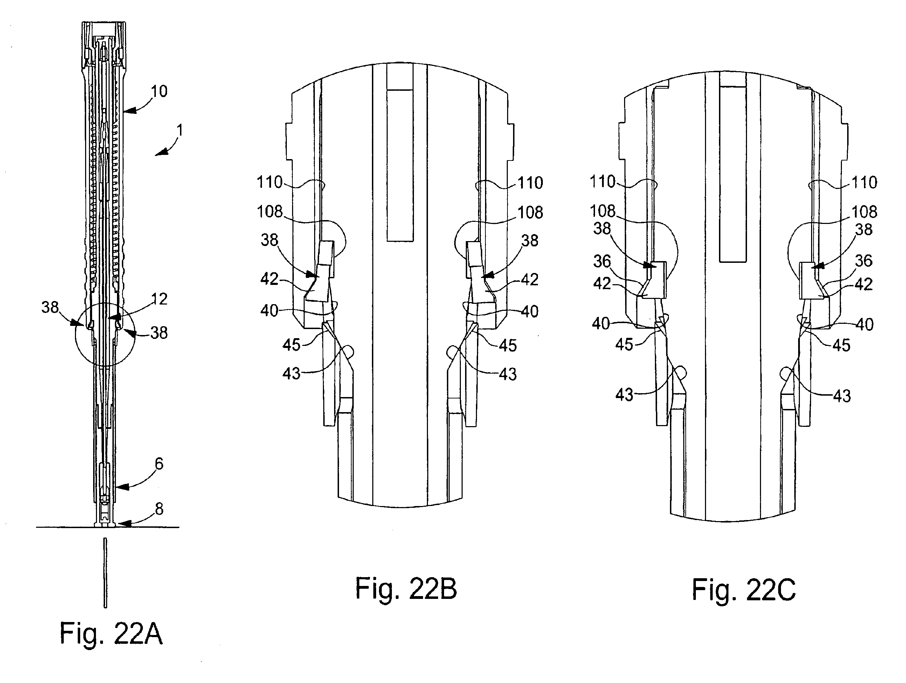

Comme illustré sur les figures 20A à 20C, le ressort 94 est en butée à son extrémité distale contre le collier 92 et à son extrémité proximale contre l'embase 58. Au fur et à mesure que la gaine 6 se rétracte à l'intérieur du corps principal 10 lorsqu'on presse le dispositif de rétro-injection 1 contre la peau 22 du sujet, le corps secondaire 12 entraîne avec lui le collier 92 qui est en appui par son chemin de came 100 contre la surface de came 98 dudit corps secondaire 12. Au cours de ce mouvement de translation, les ergots 96 du collier 92 glissent le long des rainures 90 les plus courtes des deux chemins de came 56 ménagés dans la paroi intérieure du corps principal 10. Arrivé au niveau du point de rebroussement qui est défini par l'endroit où les rainures 90 les plus courtes communiquent avec les rainures 54 les plus longues (voir figure 21A), le collier 92, qui n'est plus guidé axialement, pivote en suivant par son chemin de came 100 la surface de came 98 ménagée sur le corps secondaire 12 (voir figure 21 B). Cet évènement est concomitant avec le passage des moyens de blocage 104 du corps secondaire 12 derrière la tête 86 de la tige de piston 88. A cet instant, les ergots 96 du collier 92 pénètrent dans les rainures 54 les plus longues des chemins de came 56, autorisant le ressort 94 à se détendre à nouveau (voir figure 21 C). En se détendant, le ressort 94 pousse le corps secondaire 12 en avant, dans la direction de sortie du corps principal 10. La force de poussée du ressort 94 est transmise au corps secondaire 12 via le collier 92. Simultanément, le corps secondaire 12 pousse en avant, dans la direction de sortie du corps principal 10, la gaine 6. Le corps secondaire 12 transmet sa force de poussée à la gaine 6 par l'ntermédiaire de ses plans inclinés 40 qui butent contre le fond des bras élastiques en V 38. En effet, ces derniers, en appui contre la paroi intérieure du corps principal 10, ne sont pas autorisés à s'écarter. Le mouvement de sortie de la gaine 6 hors du corps principal 10 s'interrompt lorsque ladite gaine 6 vient en butée par ses rebords tronconiques 43 contre l'épaulement intérieur 45 prévu à l'extrémité distale du corps principal 10. Dans cette position, les ergots 42 des bras élastiques 38 se retrouvent en regard des logements 36, de sorte que lesdits bras 38 sont donc à nouveau autorisés à se déformer élastiquement vers l'extérieur du volume du corps principal 10 (voir figure 22B). Ceci autorise les plans inclinés 40 correspondants ménagés sur le corps secondaire 12 à glisser sous ces bras élastiques 38. On notera que, comme les rainures longitudinales 54 des chemins de came 56 sont plus longues que les rainures 90 dans lesquelles se déplacent les ergots 96 du collier 92 lorsque l'on enfonce l'aiguille creuse 28 dans la peau 22 du sujet, au moment où se produit la rétro-injection et où le corps secondaire 12 émerge à nouveau du corps principal 10, ledit corps secondaire 12 est autorisé à avancer légèrement au-delà de la position qu'il occupait initialement avant que l'on presse le dispositif d'injection 1 contre la peau 22. On met ce déplacement supplémentaire à profit pour faire tomber la base des bras élastiques en V 38 dans deux créneaux 108 diamétralement opposés situés entre les pieds des plans inclinés 40 et deux arêtes longitudinales 110 (voir figure 22C). De la sorte, le corps secondaire 12 est verrouillé sur la gaine 6 qui elle-même est verrouillée sur le corps principal 10, de sorte que le dispositif de rétro-injection 1 selon l'invention est totalement verrouillé après utilisation.

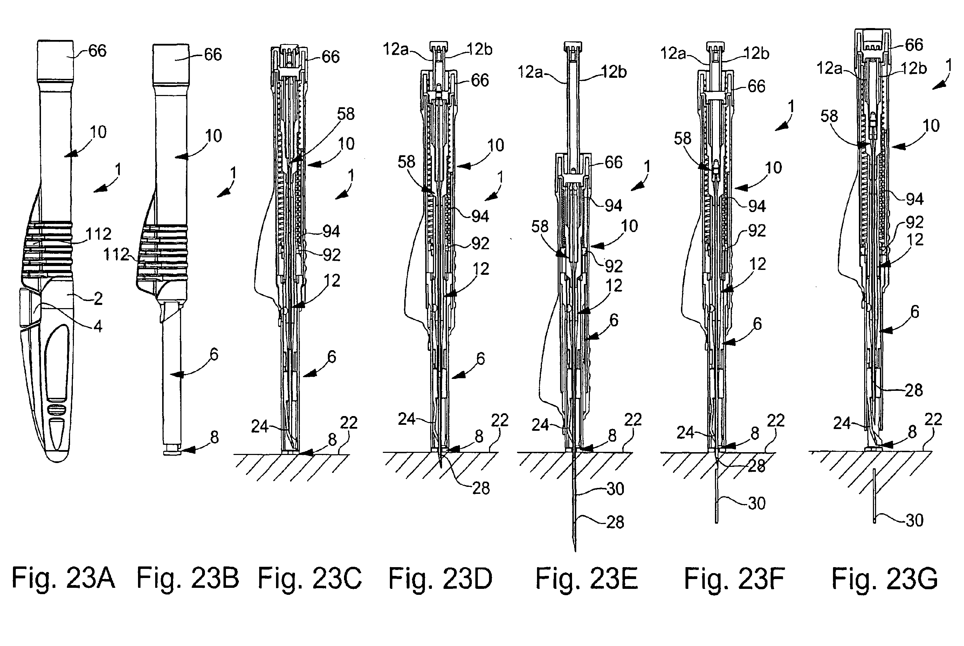

On s'intéresse maintenant en liaison avec les figures 23A à 23G au fonctionnement du dispositif de rétro-injection 1 selon l'invention. Après avoir sorti celui-ci de son emballage (figure 23A), on commence par retirer le capuchon 2 puis le cavalier 14 (figure 23B). Cette dernière opération permet de découpler la gaine 6 du corps secondaire 12. Le dispositif de rétro-injection 1 est alors prêt à l'emploi. En tenant le dispositif 1 par son corps principal 10, on l'applique contre la peau 22 du sujet (figure 23C). La présence d'une nervure striée 112 qui s'étend sur une partie de la longueur du dispositif de rétro-injection 1 assure un maintien ferme de ce dernier. On presse l'élément de retenue 8 contre la peau 22 du sujet. Sous l'effet de cette poussée, l'élément de retenue 8 pénètre légèrement à l'intérieur de la gaine 6, ce qui a pour effet d'écarter la languette élastique 24 de sa position de repos et de laisser le passage à l'aiguille creuse 28.

Simultanément, l'élément de retenue 8 pousse en arrière le corps secondaire 12 dont il est solidaire, ce qui permet aux plans inclinés 40 ménagés sur ce corps secondaire 12 de s'éloigner des bras élastiques 38 qui sont ainsi autorisés à fléchir. Par suite, les ergots 42 prévus à la base de ces bras élastiques 38 se dégagent de leur position en prise dans les logements 36 prévus à l'extrémité distale du corps principal 10, de sorte que la gaine 6 se retrouve découplée dudit corps principal 10. L'élément de retenue 8 continue de pénétrer à l'intérieur de la gaine 6 jusqu'à ce qu'il vienne en butée contre l'extrémité distale de cette dernière. L'élément de retenue 8 exerce alors une poussée sur la gaine 6 d'une part, et sur le corps secondaire 12 d'autre part, de sorte que ces deux pièces commencent à se rétracter à l'intérieur du corps principal 10. On notera que lors de ce déplacement, la position relative de la gaine 6 par rapport au corps secondaire 12 reste inchangée. La gaine 6 et le corps secondaire 12 se rétractant, l'aiguille creuse 28 émerge progressivement du corps principal 10 et pénètre dans la peau 22 du sujet (figure 23D). Le mouvement de recul du corps secondaire 12 est autorisé par le fait que les deux portions de tube 12a, 12b dont il est constitué sortent par l'extrémité proximale du dispositif de rétro-injection 1 en coulissant à l'intérieur de la douille 78, passant de part et d'autre de la barre 84 qui s'étend radialement à l'intérieur de ladite douille 78 (figure 23^{E}).

En remontant à l'intérieur du corps principal 2, le corps secondaire 12 vient à la rencontre de la tête 86 de la tige de piston 88 qui est initialement couplée avec le corps principal 10 via ses deux clips élastiques 106 pincés sur la barre 84 de la douille 78. Les deux bourrelets 104 ménagés sur la face intérieure des portions de tube 12a, 12b arrivent ainsi à hauteur de la tête 86 de la tige de piston 88 et passent derrière celle-ci en se déformant élastiquement. En parallèle, le corps secondaire 12 entraîne avec lui le collier 92 qui coulisse par ses ergots 96 le long des rainures 90 les plus courtes des chemins de came 56 ménagés de manière diamétralement opposée dans la paroi intérieure dudit corps principal 10. Le ressort 94, en appui contre le collier 92 qui remonte à l'intérieur du corps principal 10 et contre l'embase 58 qui est fixe, se comprime.

Lorsque le collier 92 arrive au niveau du point de rebroussement où les rainures 90 les plus courtes communiquent avec les rainures 54 les plus longues des chemins de came 56, les deux bourrelets 104 passent de part et d'autre de la tête 86 de la tige de piston 88. Par ailleurs, ledit collier 92 qui, à ce moment là, n'est plus guidé axialement, est forcé à pivoter sous l'effet de la pression exercée par le ressort 94 et s'engage par ses ergots 96 dans les rainures 54 les plus longues. A cet instant, le ressort 94 est à nouveau autorisé à se détendre et pousse en avant le corps secondaire 12 qui lui-même exerce une poussée sur la gaine 8 dans le sens de sortie du corps principal 10 (figure 23F). Au cours du mouvement de sortie du corps secondaire 12, les bourrelets 104 retiennent la tête 86 de la tige de piston 88 qui se découple du corps principal 10 et se retrouve couplée avec ledit corps secondaire 12. La tige de piston 88 reste ainsi immobile, pénétrant progressivement dans l'aiguille creuse 28 pour maintenir l'implant 30 en position à la bonne profondeur dans la peau 22 du sujet.

Le mouvement d'avance de la gaine 6 est interrompu lorsque celle-ci vient en butée par ses deux rebords tronconiques 43 contre l'épaulement intérieur 45 prévu à l'extrémité distale du corps principal 10. Simultanément, le corps secondaire 12 glisse par ses plans inclinés 40 sous les bras élastiques 38. Ces derniers viennent se loger dans les deux créneaux 108 prévus en arrière desdits plans inclinés 40. Le corps secondaire 12 est ainsi verrouillé sur la gaine 6 qui elle-même se verrouille sur le corps principal 10 par le biais de ses bras élastiques 38 dont les ergots 42 viennent en prise dans les logements 36 du corps principal 10. Le dispositif de rétro-injection 1 selon l'invention est ainsi totalement verrouillé et ce de manière irréversible (figure 23G).

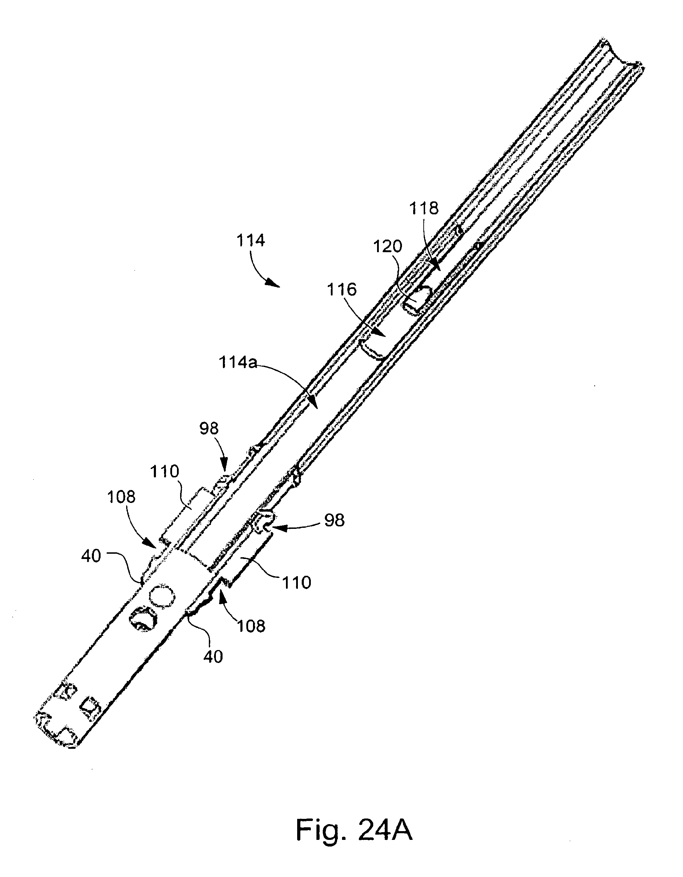

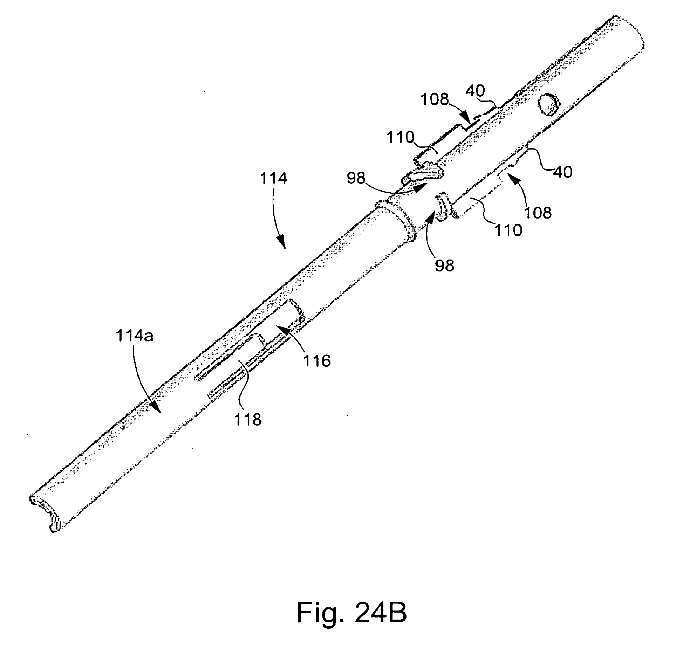

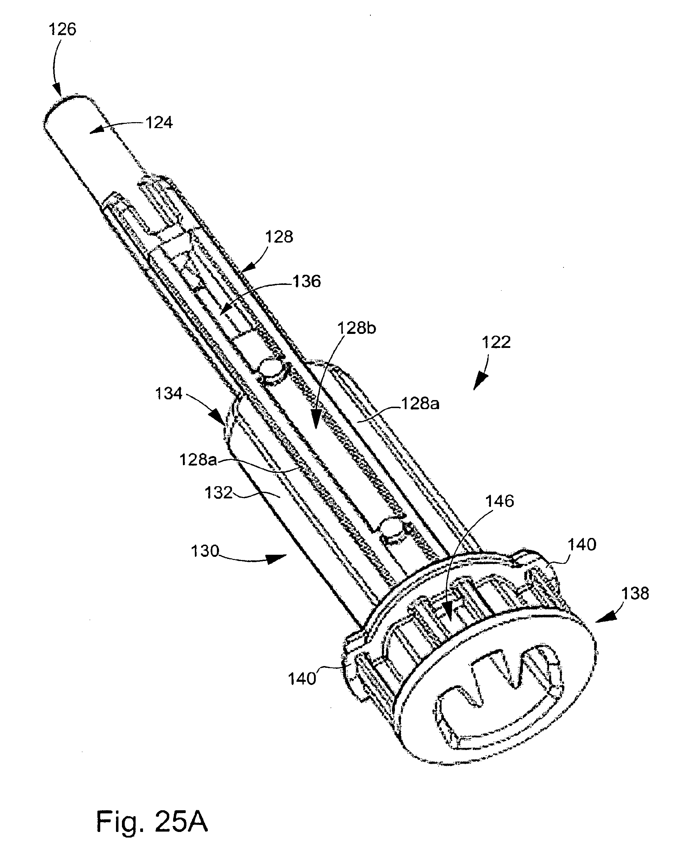

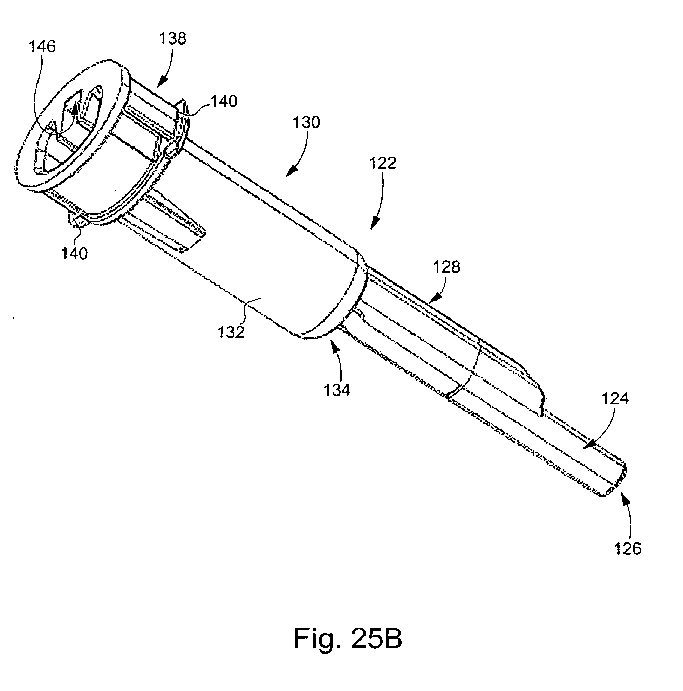

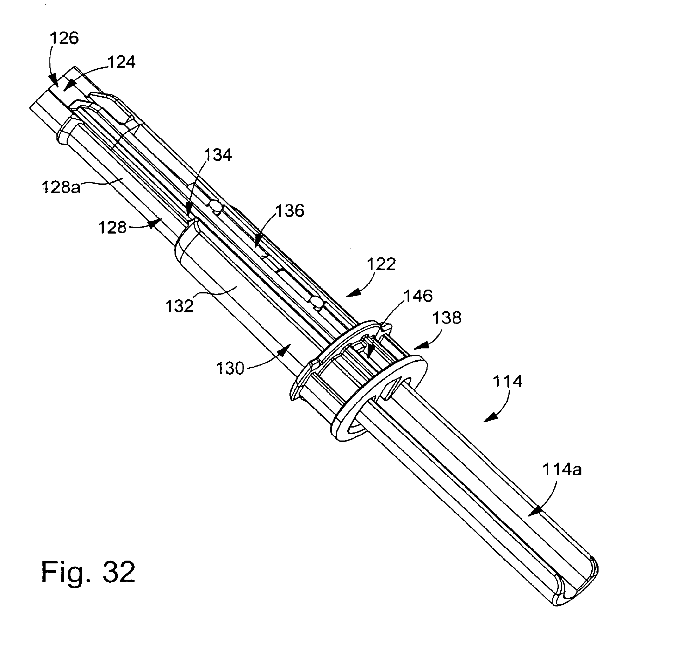

On examine maintenant une variante préférée de réalisation du dispositif de rétro-injection 1 selon l'invention. Les éléments identiques à ceux décrits en liaison avec le mode de réalisation précédent sont désignés par les mêmes références numériques et ne seront pas décrits davantage ici. Conformément à ce mode préféré de réalisation de l'invention, le corps secondaire, désigné ici par la référence numérique 114, ne comprend plus qu'une seule portion de tube 114a qui correspond sensiblement à la moitié de l'enveloppe cylindrique dans laquelle s'inscrit ledit corps secondaire 114 (voir figures 24A et 24B). On délimite dans cette portion de tube 114a une fenêtre 116 dans laquelle s'étend un bras élastique longitudinal 118 terminé à son extrémité libre par un renflement 120 orienté côté intérieur dudit tube 114a. Quant à l'embase de l'aiguille creuse 28 (voir figures 25A et 25B), désignée ici par la référence 122, elle comprend pour l'essentiel une portion de tube 124 qui définit une ouverture traversante 126 pour le maintien à friction et/ou collage de ladite aiguille 28 et qui est prolongée par une gouttière rectiligne 128 qui présente une section droite en forme de U prévue pour coopérer avec la forme du corps secondaire 114. Le corps 130 de l'embase 122 est pour sa part formé par une portion de cylindre 132 qui se rattache sur les flancs verticaux opposés 128a de la gouttière 128 de manière à délimiter un passage annulaire 134 dans lequel peut coulisser la portion de tube 114a du corps secondaire 114 (voir figure 32). On ménage d'autre part dans le fond 128b de la gouttière 128 une fenêtre 136 dont le rôle sera décrit ci-après. Enfin, le corps 130 de l'embase 122 se termine par un bouton 138 comprenant deux renflements 140 destinés à venir faire saillie dans deux ouvertures correspondantes 142 ménagées à l'extrémité proximale du corps principal 144 pour l'immobilisation de ladite embase 122 sur ledit corps principal 144. Ce faisant, le capuchon d'extrémité 66 prévu dans le premier mode de réalisation n'est plus nécessaire. On pratique également dans le bouton 138 une encoche 146 située dans le même plan et dans le prolongement de la fenêtre 136.

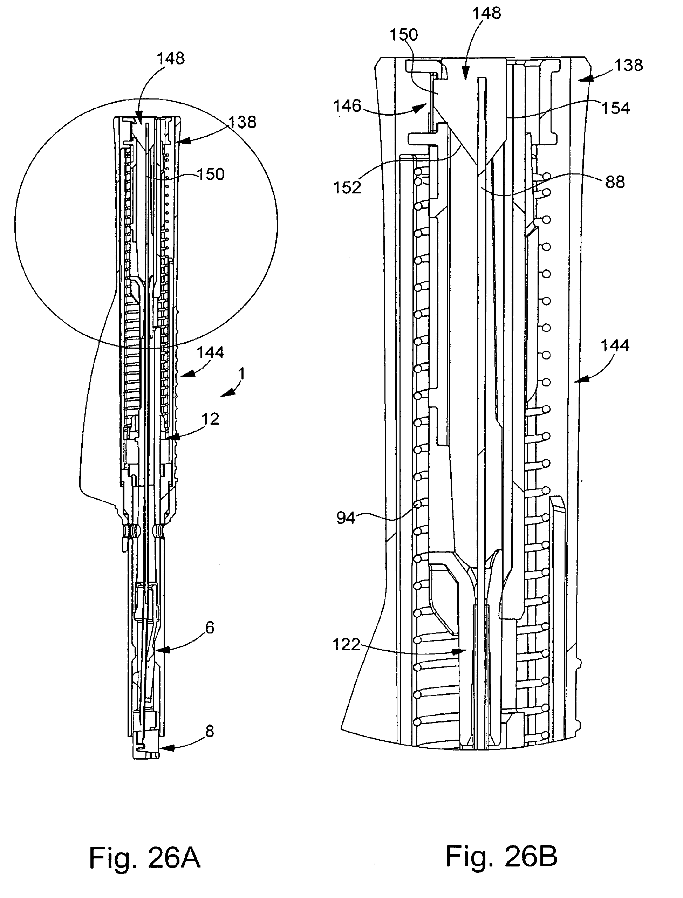

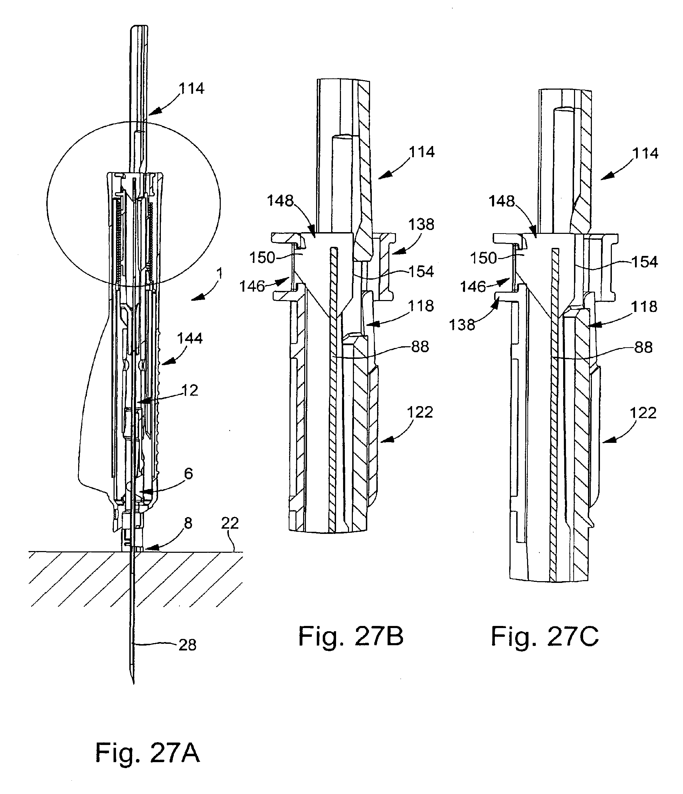

La tête 148 de la tige de piston 88 comprend un ergot 150 qui, en position de stockage du dispositif de rétro-injection 1 selon l'invention, fait saillie dans l'encoche 146 du bouton 138, ce qui assure le couplage entre ladite tige de piston 88 et le corps principal 144 via l'embase 122 (voir figure 26B). L'ergot 150 de la tête 148 de la tige de piston 88 est relié via un plan incliné 152 à un talon 154. Au fur et à mesure que l'aiguille creuse 28 pénètre dans la peau 22 du sujet, le corps secondaire 114 remonte à l'intérieur du corps principal 144 en coulissant le long de l'embase 122 (voir figure 27B). Peu de temps avant que l'aiguille 28 ne soit complètement enfoncée dans la peau 22 du sujet, la tête 148 de la tige de piston 88 glisse par son talon 154 sur le renflement 120 du bras élastique 118 qui s'écarte de sa position de repos. Pour finir, la tête 148 dépasse le renflement 120 et son talon 154 tombe dans la fenêtre 116. A ce moment là, la tête 148 de la tige de piston 88 est couplée d'une part avec le corps principal 144 et d'autre part avec le corps secondaire 114 (voir figure 27C). Le collier 92, quant à lui, change de rainure, et le ressort 94 est comprimé à son maximum.

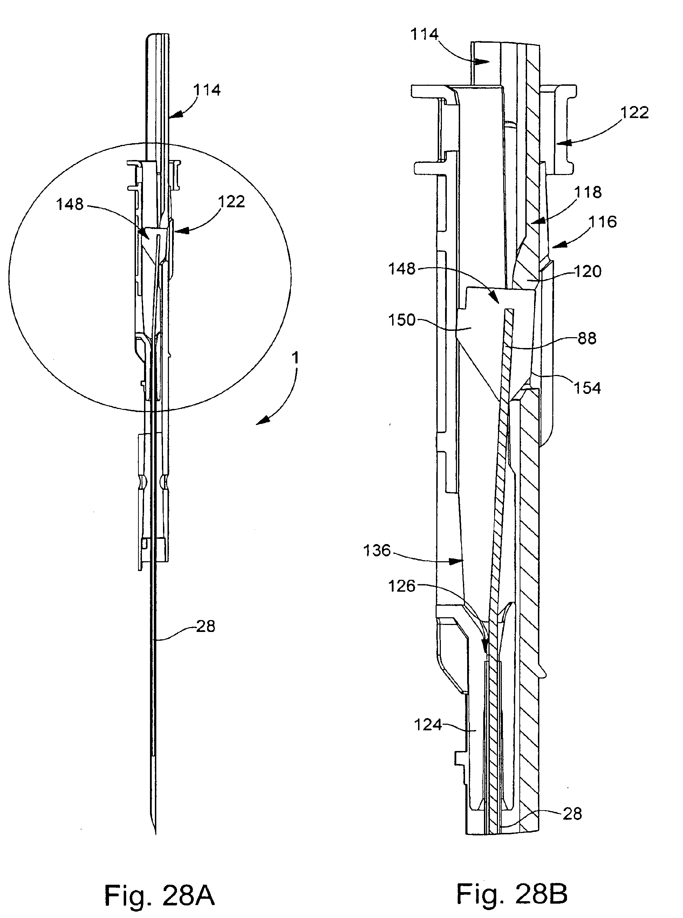

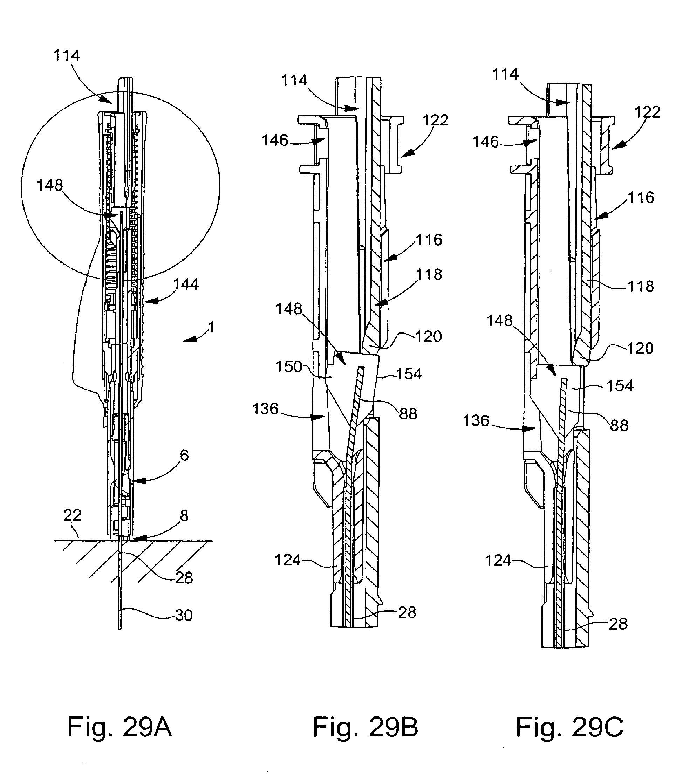

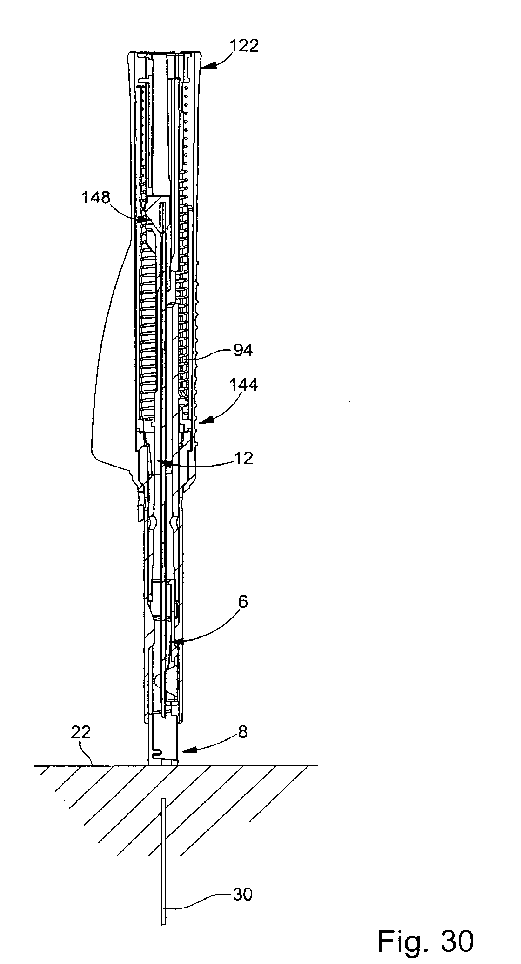

Lorsque débute la rétro-injection, le ressort 94 se détend et le corps secondaire 114 coulisse à nouveau le long de l'embase 122 mais en direction de la sortie du corps principal 144 cette fois. L'ergot 150 de la tête 148 de la tige de piston 88, retenu par le renflement 120 (voir figure 28B), se désolidarise du corps principal 144 et glisse le long du fond 128b de la gouttière 128 jusqu'au moment où il se trouve en regard de la fenêtre 136 (voir figures 29B et 29C). A cet instant, la tige de piston 88 se détend et l'ergot 150 pénètre dans ladite fenêtre 136, son talon 154 se désolidarisant du renflement 120. La tige de piston 88 se retrouve ainsi à nouveau couplée avec l'embase 122, autrement dit avec le corps principal 144, ce qui permet au corps secondaire 114 de parcourir la distance nécessaire pour le retrait complet de l'aiguille 28 hors de la peau 22 du sujet et la protection de celle-ci à l'intérieur du dispositif de rétro-injection 1 selon l'invention (voir figure 30). Dans cette position, l'implant 30 a été maintenu à la bonne profondeur dans la peau 22 du sujet, et il reste à finir de retirer l'aiguille 28 et la tige de piston 88 hors de la peau 22. Pour ce faire, la tige de piston 88, à nouveau solidaire du corps principal 144, est retirée concomitamment à l'aiguille 28.

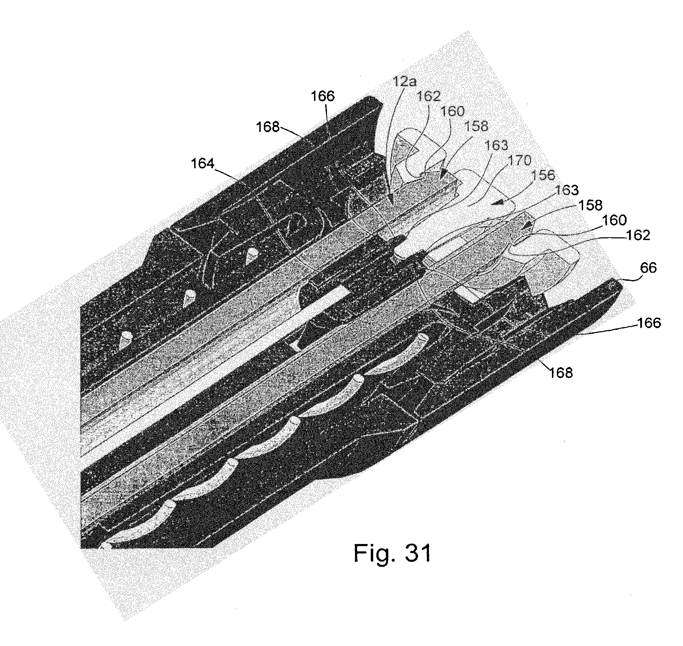

Il va de soi que la présente invention n'est pas limitée aux modes de réalisation qui viennent d'être décrits et que diverses modifications et variantes simples peuvent être envisagées par l'homme du métier sans sortir du cadre de l'invention tel qu défini par les revendications annexées. On pourrait en particulier envisager que le ressort, au lieu d'être pratiquement détendu en position de stockage du dispositif de rétro-injection, soit au contraire comprimé et se détende lors du rappel du corps secondaire hors du corps principal. Selon une variante représentée à la figure 31, les deux portions de tube 12a, 12b peuvent être maintenues ensemble par encliquetage sur un capuchon 156 tout au long de l'utilisation du dispositif de rétro-injection 1 selon l'invention. A cet effet, les deux portions de tube 12a, 12b présentent chacune un renflement 158 qui définit une surface d'accrochage 160 sur un rebord intérieur 162 du capuchon 156. Le capuchon 156 a également pour fonction d'assurer le couplage temporaire entre la tête 86 de la tige de piston 88 et le corps principal 10 en positon de stockage du dispositif de rétro-injection 1. A cet effet, la tête 86 de la tige de piston 88 se présente sous la forme d'une paire de clips 163 passés à travers une douille centrale 164 ménagée à l'intérieur du capuchon d'extrémité 66 et présentant chacun un renflement 166 définissant une surface d'accrochage 168 sur la douille 164. En position de stockage, les clips 163 sont maintenus écartés l'un de l'autre par une pointe conique 170 qui se dresse sur la surface inférieure du capuchon 156. Lorsqu'on active le dispositif de rétro-injection 1, le corps secondaire 12 sort progressivement du corps principal 10, de sorte que la pointe 170 se dégage des clips 162. Ceux-ci sont alors autorisés à se rapprocher l'un de l'autre et peuvent coulisser à l'intérieur de la douille 164, permettant ainsi le découplage de la tête 88 de la tige de piston 86 du corps principal 10.

## Revendications

1. Dispositif pour la rétro-injection d'un implant (30) dans la peau (22) d'un sujet, ce dispositif (1) comprenant un corps principal creux (10; 144) auquel est fixée une aiguille creuse (28) dans laquelle est introduit l'implant (30), un corps secondaire (12; 114) disposé coaxialement à l'intérieur du corps principal (10) et entourant l'aiguille (28), et une tige de piston (88) capable de coulisser coaxialement à l'intérieur de ladite aiguille creuse (28), des moyens étant prévus pour permettre à la tige de piston (88) de conserver inchangée sa position relativement à l'aiguille (28) lorsque le dispositif de rétro-injection (1) est pressé contre la peau (22) du sujet pour permettre à l'aiguille (28) de pénétrer dans la peau (22) dudit sujet et que le corps secondaire (12) se rétracte à l'intérieur du corps principal (10), et pour permettre à la tige de piston (88) de s'enfoncer à l'intérieur de l'aiguille creuse (28) pour maintenir l'implant (30) à la profondeur requise dans la peau (22) du sujet pendant le retrait de l'aiguille creuse (28) hors de la peau (22) du sujet, retrait au cours duquel le corps secondaire (12; 114) sort du corps principal (10), le dispositif comprenant des moyens de rappel élastiques du corps secondaire (12) hors du corps principal (10), ce dispositif étant **caractérisé en ce que** les moyens de rappel élastiques qui commandent la sortie du corps secondaire (12) hors du corps principal (10) se déclenchent automatiquement, le corps secondaire (12) venant à nouveau entourer l'aiguille creuse (28).

2. Dispositif pour la rétro-injection d'un implant selon la revendication 1, **caractérisé en ce que** les moyens de rappel élastiques comprennent un ressort hélicoïdal (94).

3. Dispositif pour la rétro-injection d'un implant selon la revendication 2, **caractérisé en ce que** le ressort (94) est interposé entre le corps principal (10) et le corps secondaire (12; 114).

4. Dispositif pour la rétro-injection d'un implant selon l'une quelconque des revendications 2 ou 3, **caractérisé en ce que** le ressort (94) se comprime pendant la phase de rétraction du corps secondaire (12; 114) à l'intérieur du corps principal (10), puis se détend lorsque l'aiguille creuse (28) a atteint sa profondeur maximale dans la peau (22) du sujet, provoquant le retour automatique du corps secondaire (12; 114) à sa position sortie hors du corps principal (10).

5. Dispositif pour la rétro-injection d'un implant selon la revendication 4, **caractérisé en ce que** le ressort (94) est en appui à l'une de ses extrémités contre un collier (92) lui-même en appui sur le corps secondaire (12; 114), et est en appui à son autre extrémité contre une embase (58; 122) qui porte l'aiguille creuse (28) et qui est solidaire du corps principal (10).

6. Dispositif pour la rétro-injection d'un implant selon la revendication 5, **caractérisé en ce que** le collier (92) comprend au moins un ergot (96) par l'intermédiaire duquel il coopère avec au moins un chemin de came (56) ménagé dans la paroi intérieure du corps principal (10), ledit collier (92) comprenant en outre un chemin de came (100) par lequel il coopère avec une surface de came (98) ménagée sur le corps secondaire (12; 114).

7. Dispositif pour la rétro-injection d'un Implant selon la revendication 6, **caractérisé en ce que** le chemin de came (56) comprend deux rainures rectilignes longitudinales (54, 90) dont l'une (54) est plus longue que l'autre (90), l'ergot (96) coulissant tout d'abord dans la rainure (90) la plus courte avant qu'il n'atteigne un point de rebroussement où les deux rainures (54, 90) communiquent entre elles et où le collier (92), qui n'est plus guidé axialement, est forcé à pivoter par le ressort (94) de façon que ledit ergot (96) pénètre dans la rainure (54) la plus longue.

8. Dispositif pour la rétro-injection d'un implant selon l'une quelconques des revendications 1 à 7, **caractérisé en ce qu'**il comprend un élément de retenue (8) destiné à empêcher l'implant (30) de tomber avant utilisation dudit dispositif (1).

9. Dispositif pour la rétro-injection d'un implant selon la revendication 8, **caractérisé en ce que** l'élément de retenue (8) comprend une languette élastique (24) qui, en position de repos, obture par une partie terminale (25) inclinée vers l'intérieur du volume de l'élément de retenue (8) le trou de passage (26) de l'aiguille creuse (28) et qui, lorsque le dispositif de rétro-injection (1) est appuyé contre la peau (22) du sujet, s'écarte pour libérer le passage de ladite aiguille creuse (28).

10. Dispositif pour la rétro-injection d'un implant selon la revendication 8, **caractérisé en ce que** l'élément de retenue (8) comprend une languette élastique (46) au-dessus de laquelle passe l'aiguille creuse (28) et qui, en position de stockage du dispositif de rétro-injection (1), est fléchie vers l'intérieur du volume dudit élément de retenue (8), de sorte que l'aiguille (28) est écartée de sa direction générale d'avance, la languette élastique (46) recouvrant sa position de repos dans laquelle elle autorise ladite aiguille (28) à se réaligner et à avancer lorsque l'on presse ledit dispositif de rétro-injection (1) contre la peau (22) du sujet.

11. Dispositif pour la rétro-injection d'un implant selon l'une quelconque des revendications 1 à 10, **caractérisé en ce qu'**il comprend en outre une gaine (6) qui coopère avec le corps secondaire (12; 114) pour permettre le verrouillage irréversible du dispositif de rétro-injection (1) après utilisation.

12. Dispositif pour la rétro-injection d'un implant selon la revendication 11, **caractérisé en ce que** le corps secondaire (12; 114) se verrouille sur la gaine (6) qui elle-même se verrouille sur le corps principal (1).

13. Dispositif pour la rétro-injection d'un implant selon la revendication 12, **caractérisé en ce que** la gaine (6) comprend au moins un bras élastique (38) qui, après utilisation du dispositif de rétro-injection (1), est écarté de sa position de repos par le corps secondaire (12, 114) et vient se loger par son extrémité libre dans un créneau (108) prévu sur ledit corps secondaire (12; 114), le bras élastique (38) comprenant en outre à son extrémité libre un ergot (42) qui vient faire saillie dans un logement (36) ménagé à l'extrémité distale du corps principal (10).

14. Dispositif pour la rétro-injection d'un implant selon la revendication 13, **caractérisé en ce que** la gaine (6) assure également le verrouillage temporaire du dispositif de rétro-injection (1) avant utilisation.

15. Dispositif pour la rétro-injection d'un implant selon la revendication 14, **caractérisé en ce que** le corps secondaire (12; 114) présente sur son pourtour extérieur un plan incliné (40) par lequel il maintient le bras élastique (38) dans le logement (36) en position de stockage du dispositif de rétro-injection (1).

16. Dispositif pour la rétro-injection d'un implant selon l'une quelconque des revendications 11 à 15 en ce qu'elles dépendent de l'une quelconque des revendications 9 ou 10, **caractérisé en ce que** la gaine (6) coopère avec l'élément de retenue (8) pour libérer le passage à l'aiguille creuse (28).

17. Dispositif pour la rétro-injection d'un implant selon la revendication 16, **caractérisé en ce que** la gaine (5) présente sur le pourtour intérieur de son extrémité distale au moins un plan incliné (32) qui vient appuyer sur la languette élastique (24) lorsque l'on presse le dispositif de rétro-injection (1) contre la peau (22) du sujet, de façon que ladite languette (24) s'écarte de sa position de repos et libère le passage à l'aiguille (28), ou un ergot (48) qui, en position de stockage du dispositif de rétro-injection (1), fléchit la languette élastique (46) vers l'intérieur du volume de l'élément de retenue (8), cet ergot (48) s'éloignant de ladite languette élastique (46) qui recouvre sa position de repos et autorise l'aiguille (28) à s'aligner avec son axe général d'avance lorsque l'on presse ledit dispositif de rétro-injection (1) contre la peau (22) du sujet.

18. Dispositif pour la rétro-injection d'un implant selon la revendication 17, **caractérisé en ce que** la gaine (6) comprend deux plans inclinés (32) entre lesquels vient glisser une nervure (34) prévue sur l'élément de retenue (8) pour l'indexation de la position dudit élément de retenue (8) à l'intérieur de ladite gaine (6).

19. Dispositif pour la rétro-injection d'un implant selon la revendication 17, **caractérisé en ce que** l'élément de retenue (8) comprend une rainure dans laquelle glisse l'ergot (48) pour l'indexation de la position dudit élément de retenue (8) relativement à la gaine (6).

20. Dispositif pour la rétro-injection d'un implant selon l'une quelconque des revendications 11 à 19 en ce qu'elles dépendent de l'une quelconque des revendications 6 ou 7, **caractérisé en ce que** la position de la gaine (6) relativement au corps principal (10) est indexée par au moins un ergot (52) qui est engagé dans la rainure (54) la plus longue du chemin de came (56).

21. Dispositif pour la rétro-injection d'un implant selon l'une quelconque des revendications 5 à 20, **caractérisé en ce que** l'embase (58) comprend une portion de tube (72) qui définit une ouverture traversante (74) pour le maintien de l'aiguille creuse (24) et qui est reliée au corps cylindrique creux (53) de ladite embase (58) par au moins une nervure (76).

22. Dispositif pour la rétro-injection d'un implant selon la revendication 21, **caractérisé en ce que** l'embase (58) est coiffée par un capuchon (66) à l'intérieur duquel s'étend une douille (78), une barre (84) s'étendant diamétralement à l'intérieur de cette douille (78).

23. Dispositif pour la rétro-injection d'un implant selon la revendication 22, **caractérisé en ce que** le corps secondaire (12) est un corps creux de forme sensiblement cylindrique pourvu de deux fentes rectilignes (102) diamétralement opposées qui s'étendent depuis l'extrémité proximale dudit corps secondaire (12) jusqu'à une hauteur (h) au-dessus de l'extrémité distale (4) du dispositif de rétro-injection (1), ces fentes (102) matérialisant deux portions de tube (12a, 12b) qui passent de part et d'autre de la portion de tube (72), pénètrent à l'intérieur du corps cylindrique creux (59) de l'embase (58), puis passent de part et d'autre de la barre (84) avant de pénétrer à l'intérieur de la douille (78).

24. Dispositif de rétro-injection d'un implant selon la revendication 23, **caractérisé en ce que** les deux portions de tube (12a, 12b) comprennent des moyens de blocage (104).

25. Dispositif de rétro-injection d'un implant selon la revendication 24, **caractérisé en ce que** la tige de piston (88) comprend une tête (86) par laquelle elle est couplée avec la barre (84) pour être entraînée par le corps principal (10) lorsque l'aiguille (28) s'enfonce dans la peau (22) du sujet, et par laquelle elle coopère avec les moyens de blocage (104) pour se découpler dudit corps principal (10) et se coupler avec le corps secondaire (12) lorsque l'aiguille (28) est retirée de la peau (22) du sujet.

26. Dispositif de rétro-injection d'un implant selon l'une quelconque des revendications 5 à 20, **caractérisé en ce que** l'embase (122) comprend une portion de tube (124) qui définit une ouverture traversante (126) pour le maintien de l'aiguille creuse (28) et qui est prolongée par une gouttière rectiligne (128) sur les flancs opposés (128a) de laquelle se rattache une portion de cylindre (132) de manière à délimiter un passage annulaire (134), une fenêtre (136) étant ménagée dans le fond (128b) de ladite gouttière (128), l'embase (122) comprenant en outre à son extrémité proximale un bouton (138) dans lequel est pratiquée une encoche (146).

27. Dispositif de rétro-injection d'un implant selon la revendication 26, **caractérisé en ce que** le corps secondaire (114) comprend une seule portion de tube (114a) qui est capable de coulisser dans l'espace annulaire (134), un bras élastique (118) terminé à son extrémité libre par un renflement (120) s'étendant dans une fenêtre (116) ménagée dans la portion de tube (114a).

28. Dispositif de rétro-injection d'un implant selon la revendication 27, **caractérisé en ce que** la tige de piston (88) comprend une tête (148) par laquelle elle est couplée avec le bouton (138) pour être entraînée par le corps principal (10) lorsque l'aiguille (28) s'enfonce dans la peau (22) du sujet, et par laquelle elle coopère avec le corps secondaire (114) pour rester immobile lorsque l'aiguille (28) est retirée de la peau (22) du sujet.

29. Dispositif de rétro-injection d'un implant selon la revendications 28, **caractérisé en ce que** la tête (148) de la tige de piston (88) comprend un ergot (150) qui, en position de stockage du dispositif de rétro-injection (1), fait saillie dans l'encoche (146) du bouton (138), l'ergot (150) étant relié via un plan incliné (152) à un talon (154) par lequel ledit ergot (150) glisse sur le renflement (120) du bras élastique (118) et finit par le dépasser pour tomber dans la fenêtre (116) au moment où l'aiguille (28) est complètement enfoncée dans la peau (22) du sujet, l'ergot (150) étant par suite retenu par le renflement (120), ce qui lui permet de se découpler du corps principal (144) au fur et à mesure que le corps secondaire (114) sort à nouveau dudit corps principal (144), ce jusqu'au moment où ledit ergot (150) se trouve en regard de la fenêtre (136) et y pénètre, son talon (154) se découplant du renflement (120), de sorte que la tige de piston (88) se retrouve à nouveau couplée avec le corps principal (144), ce qui permet au corps secondaire (114) de parcourir la distance nécessaire au retrait complet de l'aiguille (28) hors de la peau (22) du sujet.

## Patentansprüche

1. Vorrichtung für die Retroinjektion eines Implantats (30) in die Haut (22) eines Lebewesens, wobei diese Vorrichtung (1) einen hohlen Hauptkörper (10; 144), an dem eine Hohlnadel (28) befestigt ist, in den das Implantat (30) eingeführt ist, einen sekundären Körper (12; 114), der koaxial in dem Hauptkörper (10) angeordnet ist und die Nadel (28) umgibt, und eine Kolbenstange (88), die in der Hohlnadel (28) koaxial gleiten kann, umfasst, wobei Mittel vorgesehen sind, um der Kolbenstange (88) zu ermöglichen, ihre Position in Bezug auf die Nadel (28) unverändert zu lassen, wenn die Retroinjektionsvorrichtung (1) gegen die Haut (22) des Lebewesens gepresst wird, um der Nadel (28) zu ermöglichen, in die Haut (22) des Lebewesens einzudringen, und der sekundäre Körper (12) sich in den Hauptkörper (10) zurückzieht, und um der Kolbenstange (88) zu ermöglichen, in die Hohlnadel (28) einzudringen, um das Implantat (30) während des Zurückziehens der Hohlnadel (28) aus der Haut (22) des Lebewesens auf der erforderlichen Tiefe in der Haut (22) des Lebewesens zu halten, wobei der sekundäre Körper (12; 114) während des Zurückziehens den Hauptkörper (10) verlässt, wobei die Vorrichtung Mittel zum elastischen Zurückstellen des sekundären Körpers (12) aus dem Hauptkörper (10) umfasst, wobei diese Vorrichtung **dadurch gekennzeichnet ist, dass** die elastischen Rückstellmittel, die den Austritt des sekundären Körpers (12) aus dem Hauptkörper (10) steuern, automatisch ausgelöst werden, wobei der sekundäre Körper (12) erneut die Hohlnadel (28) umgibt.

2. Vorrichtung für die Retroinjektion eines Implantats nach Anspruch 1, **dadurch gekennzeichnet, dass** die elastischen Rückstellmittel eine Schraubenfeder (94) umfassen.

3. Vorrichtung für die Retroinjektion eines Implantats nach Anspruch 2, **dadurch gekennzeichnet, dass** die Feder (94) zwischen den Hauptkörper (10) und den sekundären Körper (12; 114) eingefügt ist.

4. Vorrichtung für die Retroinjektion eines Implantats nach einem der Ansprüche 2 oder 3, **dadurch gekennzeichnet, dass** die Feder (94) während der Phase des Zurückziehens des sekundären Körpers (12; 114) in dem Hauptkörper (10) komprimiert wird, und sich dann entspannt, wenn die Hohlnadel (28) ihre maximale Tiefe in der Haut (22) des Lebewesens erreicht hat, was die automatische Rückkehr des sekundären Körpers (12; 114) in seine Austrittsposition aus dem Hauptkörper (10) hervorruft.

5. Vorrichtung für die Retroinjektion eines Implantats nach Anspruch 4, **dadurch gekennzeichnet, dass** sich die Feder (94) an einem ihrer Enden an einem Kranz (92) abstützt, der sich seinerseits an dem sekundären Körper (12; 114) abstützt, und sich an ihrem anderen Ende an einem Sockel (58; 122) abstützt, der die Hohlnadel (28) trägt und der mit dem Hauptkörper (10) fest verbunden ist.

6. Vorrichtung für die Retroinjektion eines Implantats nach Anspruch 5, **dadurch gekennzeichnet, dass** der Kranz (92) wenigstens einen Ansatz (96) umfasst, über den er mit wenigstens einer Nockenkurve (56) zusammenwirkt, die in der Innenwand des Hauptkörpers (10) ausgebildet ist, wobei der Kranz (92) außerdem eine Nockenkurve (100) aufweist, über die er mit einer Nockenoberfläche (98) zusammenwirkt, die in dem sekundären Körper (12; 114) ausgebildet ist.

7. Vorrichtung für die Retroinjektion eines Implantats nach Anspruch 6, **dadurch gekennzeichnet, dass** die Nockenkurve (56) zwei longitudinale geradlinige Rillen (54, 90) umfasst, wovon eine (54) länger als die andere (90) ist, wobei der Ansatz (96) zunächst in der kürzesten Rille (90) gleitet, bevor er einen Umkehrpunkt erreicht, an dem die zwei Rillen (54, 90) miteinander kommunizieren, und an dem der Kranz (92), der axial nicht mehr geführt wird, dazu gezwungen wird, durch die Feder (94) zu schwenken, derart, dass der Ansatz (96) in die längste Rille (54) eindringt.

8. Vorrichtung für die Retroinjektion eines Implantats nach einem der Ansprüche 1 bis 7, **dadurch gekennzeichnet, dass** sie ein Halteelement (8) umfasst, das dazu bestimmt ist, zu verhindern, dass das Implantat (30) vor der Verwendung der Vorrichtung (1) herabfällt.

9. Vorrichtung für die Retroinjektion eines Implantats nach Anspruch 8, **dadurch gekennzeichnet, dass** das Halteelement (8) eine elastische Zunge (24) umfasst, die in der Ruhestellung durch einen Abschlussteil (25), der in das Volumen des Halteelements (8) geneigt ist, das Durchgangsloch (26) der Hohlnadel (28) verschließt und dass, wenn die Retroinjektionsvorrichtung (1) gegen die Haut (22) des Lebewesens gedrückt wird, sich entfernt, um den Durchgang der Hohlnadel (28) freizugeben.

10. Vorrichtung für die Retroinjektion eines Implantats nach Anspruch 8, **dadurch gekennzeichnet, dass** das Halteelement (8) eine elastische Zunge (46) umfasst, über der sich die Hohlnadel (28) bewegt und die in der Position für die Aufbewahrung der Retroinjektionsvorrichtung (1) in das Volumen des Halteelements (8) gebogen ist, derart, dass die Nadel (28) aus ihrer allgemeinen Vorwärtsbewegungsrichtung entfernt ist, wobei die elastische Zunge (46) wieder ihre Ruheposition einnimmt, in der sie zulässt, dass sich die Nadel (28) wieder ausrichtet und vorwärts bewegt, wenn die Retroinjektionsvorrichtung (1) gegen die Haut (22) des Lebewesens gepresst wird.

11. Vorrichtung für die Retroinjektion eines Implantats nach einem der Ansprüche 1 bis 10, **dadurch gekennzeichnet, dass** sie außerdem eine Hülle (6) umfasst, die mit dem sekundären Körper (12; 114) zusammenwirkt, um die unumkehrbare Verriegelung der Retroinjektionsvorrichtung (1) nach der Verwendung zu erlauben.

12. Vorrichtung für die Retroinjektion eines Implantats nach Anspruch 11, **dadurch gekennzeichnet, dass** der sekundäre Körper (12; 114) an der Hülle (6) verriegelt, die ihrerseits an dem Hauptkörper (1) verriegelt.

13. Vorrichtung für die Retroinjektion eines Implantats nach Anspruch 12, **dadurch gekennzeichnet, dass** die Hülle (6) wenigstens einen elastischen Arm (38) umfasst, der nach der Verwendung der Retroinjektionsvorrichtung (1) durch den sekundären Körper (12, 114) aus seiner Ruhestellung entfernt wird und mit seinem freien Ende in eine in dem sekundären Körper (12; 114) vorgesehene Vertiefung (108) eintritt, wobei der elastische Arm (38) außerdem an seinem freien Ende einen Ansatz (42) aufweist, der in einen Aufnahmesitz (36) vorsteht, der am distalen Ende des Hauptkörpers (10) ausgebildet ist.

14. Vorrichtung für die Retroinjektion eines Implantats nach Anspruch 13, **dadurch gekennzeichnet, dass** die Hülle (6) außerdem die vorübergehende Verriegelung der Retroinjektionsvorrichtung (1) vor der Verwendung sicherstellt.

15. Vorrichtung für die Retroinjektion eines Implantats nach Anspruch 14, **dadurch gekennzeichnet, dass** der sekundäre Körper (12; 114) an seinem äußeren Umfang eine geneigte Ebene (40) aufweist, durch die er den elastischen Arm (38) in dem Aufnahmesitz (36) in der Aufbewahrungsposition der Retroinjektionsvorrichtung (1) hält.

16. Vorrichtung für die Retroinjektion eines Implantats nach einem der Ansprüche 11 bis 15, wenn abhängig von einem der Ansprüche 9 oder 10, **dadurch gekennzeichnet, dass** die Hülle (6) mit dem Halteelement (8) zusammenwirkt, um den Durchgang der Hohlnadel (28) freizugeben.

17. Vorrichtung für die Retroinjektion eines Implantats nach Anspruch 16, **dadurch gekennzeichnet, dass** die Hülle (6) an ihrem inneren Umfang ihres distalen Endes wenigstens eine geneigte Ebene (32) aufweist, die sich an der elastischen Zunge (24) abstützt, wenn die Retroinjektionsvorrichtung (1) gegen die Haut (22) des Lebewesens gepresst wird, derart, dass sich die Zunge (24) aus ihrer Ruheposition entfernt und den Durchgang der Nadel (28) freigibt, oder einen Ansatz (48) aufweist, der in der Aufbewahrungsposition der Retroinjektionsvorrichtung (1) die elastische Zunge (46) in das Volumen des Halteelements (8) biegt, wobei sich dieser Ansatz (48) von der elastischen Zunge (46), die wieder ihre Ruheposition einnimmt, entfernt und zulässt, dass sich die Nadel (28) auf ihre allgemeine Vorwärtsbewegungsachse ausrichtet, wenn die Retroinjektionsvorrichtung (1) gegen die Haut (22) des Lebewesens gepresst wird.

18. Vorrichtung für die Retroinjektion eines Implantats nach Anspruch 17, **dadurch gekennzeichnet, dass** die Hülle (6) zwei geneigte Ebenen (32) aufweist, zwischen denen eine Rippe (34) gleitet, der an dem Halteelement (8) vorgesehen ist, um die Position des Halteelements (8) in der Hülle (6) zu indexieren.

19. Vorrichtung für die Retroinjektion eines Implantats nach Anspruch 17, **dadurch gekennzeichnet, dass** das Halteelement (8) eine Rille aufweist, in der der Ansatz (48) zum Indexieren der Position des Halteelements (8) in Bezug auf die Hülle (6) gleitet.

20. Vorrichtung für die Retroinjektion eines Implantats nach einem der Ansprüche 11 bis 19, wenn abhängig von einem der Ansprüche 6 oder 7, **dadurch gekennzeichnet, dass** die Position der Hülle (6) in Bezug auf den Hauptkörper (10) durch wenigstens einen Ansatz (52) indexiert ist, der in der längsten Rille (54) der Nockenkurve (56) in Eingriff ist.

21. Vorrichtung für die Retroinjektion eines Implantats nach einem der Ansprüche 5 bis 20, **dadurch gekennzeichnet, dass** der Sockel (58) einen Rohrabschnitt (72) aufweist, der eine Durchgangsöffnung (74) zum Halten der Hohlnadel (24) definiert und der mit dem zylindrischen Hohlkörper (53) des Sockels (58) über wenigstens eine Rippe (76) verbunden ist.

22. Vorrichtung für die Retroinjektion eines Implantats nach Anspruch 21, **dadurch gekennzeichnet, dass** der Sockel (58) von einer Kappe (66) überragt wird, in der sich eine Buchse (78) erstreckt, wobei sich in dieser Buchse (78) ein Stab (84) diametral erstreckt.

23. Vorrichtung für die Retroinjektion eines Implantats nach Anspruch 22, **dadurch gekennzeichnet, dass** der sekundäre Körper (12) ein Hohlkörper mit im Wesentlichen zylindrischer Form ist, der mit zwei diametral gegenüberliegenden geradlinigen Schlitzen (102) versehen ist, die sich von dem proximalen Ende des sekundären Körpers (12) bis zu einer Höhe (h) über dem distalen Ende (4) der Retroinjektionsvorrichtung (1) erstrecken, wobei diese Schlitze (102) zwei Rohrabschnitte (12a, 12b) ergeben, die beiderseits des Rohrabschnitts (72) verlaufen, dann in den zylindrischen Hohlkörper (59) des Sockels (58) eindringen und dann beiderseits des Stabs (84) verlaufen, bevor sie in die Buchse (78) eindringen.

24. Vorrichtung für die Retroinjektion eines Implantats nach Anspruch 23, **dadurch gekennzeichnet, dass** die zwei Rohrabschnitte (12a, 12b) Blockiermittel (104) umfassen.

25. Vorrichtung für die Retroinjektion eines Implantats nach Anspruch 24, **dadurch gekennzeichnet, dass** die Kolbenstange (88) einen Kopf (86) aufweist, über den sie mit der Stange (84) gekoppelt ist, um durch den Hauptkörper (10) angetrieben zu werden, wenn die Nadel (28) in die Haut (22) des Lebewesens eindringt, und durch den sie mit den Blockiermitteln (104) zusammenwirkt, um sich von dem Hauptkörper (10) zu entkoppeln und um mit dem sekundären Körper (12) zu koppeln, wenn die Nadel (28) aus der Haut (22) des Lebewesens zurückgezogen wird.

26. Vorrichtung für die Retroinjektion eines Implantats nach einem der Ansprüche 5 bis 20, **dadurch gekennzeichnet, dass** der Sockel (122) einen Rohrabschnitt (124) aufweist, der eine Durchgangsöffnung (126) definiert, um die Hohlnadel (28) zu halten, und der durch eine geradlinige Rinne (128) verlängert ist, an deren gegenüberliegenden Seitenflächen (128a) ein zylindrischer Abschnitt (132) angefügt ist, derart, dass ein ringförmiger Durchgang (134) begrenzt wird, wobei in dem Boden (128b) der Rinne (128) ein Fenster (136) ausgebildet ist, wobei der Sockel (122) außerdem an seinem proximalen Ende eine Taste (138) aufweist, in der eine Kerbe (146) ausgespart ist.

27. Vorrichtung für die Retroinjektion eines Implantats nach Anspruch 26, **dadurch gekennzeichnet, dass** der sekundäre Körper (114) einen einzigen Rohrabschnitt (114a) aufweist, der in dem ringförmigen Raum (134) gleiten kann, wobei sich ein elastischer Arm (118), der an seinem freien Ende in einer Verdickung (120) endet, in einem Fenster (116) erstreckt, das in dem Rohrabschnitt (114a) ausgebildet ist.

28. Vorrichtung für die Retroinjektion eines Implantats nach Anspruch 27, **dadurch gekennzeichnet, dass** die Kolbenstange (88) einen Kopf (148) aufweist, über den sie mit der Taste (138) gekoppelt ist, um durch den Hauptkörper (10) angetrieben zu werden, wenn die Nadel (28) in die Haut (22) des Lebewesens eindringt, und über den sie mit dem sekundären Körper (114) zusammenwirkt, um unbeweglich zu bleiben, wenn die Nadel (28) aus der Haut (22) des Lebewesens zurückgezogen wird.

29. Vorrichtung für die Retroinjektion eines Implantats nach Anspruch 28, **dadurch gekennzeichnet, dass** der Kopf (148) der Kolbenstange (88) einen Ansatz (150) aufweist, der in der Aufbewahrungsposition der Retroinjektionsvorrichtung (1) in die Kerbe (146) der Taste (138) vorsteht, wobei der Ansatz (150) über eine geneigte Ebene (152) mit einer Nase (154) verbunden ist, durch die der Ansatz (150) an der Verdickung (120) des elastischen Arms (118) gleitet und am Ende überschreitet er sie, um zu dem Zeitpunkt, zu dem die Nadel (28) vollständig in die Haut (22) des Lebewesens eingedrungen ist, in das Fenster (116) zu fallen, wobei der Ansatz (150) anschließend durch die Verdickung (120) gehalten wird, was ihm ermöglicht, von dem Hauptkörper (144) nach Maßgabe des erneuten Austritts des sekundären Körpers (114) aus dem Hauptkörper (144) zu ent-koppeln, bis zu dem Zeitpunkt, zu dem der Ansatz (150) sich gegenüber dem Fenster (136) befindet und darin eindringt, wobei seine Nase (154) von der Verdickung (120) entkoppelt, derart, dass die Kolbenstange (88) erneut mit dem Hauptkörper (144) koppelt, was dem sekundären Körper (114) ermöglicht, die Strecke zu durchlaufen, die erforderlich ist, um die Nadel (28) vollständig aus der Haut (22) des Lebewesens zurückzuziehen.

## Claims

1. Device for back injecting an implant (30) into the skin (22) of a subject, said device (1) including a hollow main body (10; 144) to which a hollow needle (28) is fixed, into which the implant (30) is introduced, a secondary body (12; 114) coaxially arranged inside the main body (10) and surrounding the needle (28), and a piston rod (88) capable of sliding coaxially inside said hollow needle (28), means being provided to allow the piston rod (88) to keep unchanged the position thereof relative to the needle (28) when the back injection device (1) is pressed against the subject's skin (22) to allow the needle (28) to penetrate said subject's skin (22) and when the secondary body (12) retracts inside the main body (10), and to allow the piston rod (88) to move inside the hollow needle (28) to hold the implant (30) at the required depth in the subject's skin (22) while the hollow needle (28) is being withdrawn from the subject's skin (22), retraction during which the secondary body (12; 114) exits the main body (10), the device including means for elastically returning the secondary body (12) out of the main body (10), the device being **characterized in that** the elastic return means which control the exit of the secondary body (12) from the main body (10) are triggered automatically and the secondary body (12) again surrounds the hollow needle (22).

2. Implant back injection device according to claim 1, **characterized in that** the elastic return means include a helical spring (94).

3. Implant back injection device according to claim 2, **characterized in that** the spring (94) is inserted between the main body (10) and the secondary body (12; 114).

4. Implant back injection device according to any of claims 2 or 3, **characterized in that** the spring (94) compresses during the phase of retraction of the secondary body (12; 114) inside the main body (10), then is let down when the hollow needle (28) has reached the maximum depth thereof in the subject's skin (22), causing the automatic return of the secondary body (12; 114) to the exit position thereof out of the main body (10).

5. Implant back injection device according to claim 4, **characterized in that** the spring (94) abuts at one of the ends thereof against a collar (92) which itself abuts the secondary body (12; 114) and whose other end abuts against a base (58; 122) which carries the hollow needle (28) and which is secured to the main body (10).

6. Implant back injection device according to claim 5, **characterized in that** the collar (92) includes at least one snug (96) via which it cooperates with at least one cam path (56) arranged in the inner wall of the main body (10), said collar (92) further including a cam path (100) via which it cooperates with a cam surface (98) arranged on the secondary body (12; 114).

7. Implant back injection device according to claim 6, **characterized in that** the cam path (56) includes two longitudinal rectilinear grooves (54, 90) one (54) of which is longer than the other (90), the snug (96) sliding first of all into the shorter groove (90) prior to reaching a point of return where the two grooves (54, 90) communicate with each other and where the collar (92), which is no longer guided axially, is forced to pivot via the spring (94) such that said snug (96) penetrates the longer groove (54).

8. Implant back injection device according to any of claims 1 to 7, **characterized in that** it includes a retaining element (8) for preventing the implant (30) from falling prior to use of said device (1).

9. Implant back injection device according to claim 8, **characterized in that** the retaining element (8) includes an elastic tongue (24), which, in the rest position, seals the through hole (26) for the hollow needle (28) via an end part (25) inclined towards the interior of the volume of the retaining element (8), and which, when the back injecting device (1) is pressed against the subject's skin (22), moves away to free the passage for said hollow needle (28).

10. Implant back injection device according to claim 8, **characterized in that** the retaining element (8) includes an elastic tongue (46) above which the hollow needle (28) passes and which, in the storage position of the back injection device (1), is bent towards the interior of the volume of said retaining element (8), such that the needle (28) is moved away from the general direction of forward movement thereof, the elastic tongue (46) returning to the rest position in which it allows said needle (28) to be realigned and move forward when said back injection device (1) is pressed against the subject's skin (22).

11. Implant back injection device according to any of claims 1 to 10, **characterized in that** it further includes a sheath (6) which cooperates with the secondary body (12; 114) for irreversibly locking the back injection device (1) after use.

12. Implant back injection device according to claim 11, **characterized in that** the secondary body (12; 114) locks onto the sheath (6) which itself locks onto the main body (10).

13. Implant back injection device according to claim 12, **characterized in that** the sheath (6) includes at least one elastic arm (38), which, after the back injection device (1) has been used, is moved away from the rest position thereof by the secondary body (12, 114) and is housed via the free end thereof in a slot (108) provided on said secondary body (12; 114), the elastic arm (38) further including at the free end thereof a snug (42), which projects into a housing (36) arranged at the distal end of the main body (10).

14. Implant back injection device according to claim 13, **characterized in that** the sheath (6) also temporarily locks the back injection device (1) prior to use.

15. Implant back injection device according to claim 14, **characterized in that** the secondary body (12; 114) has on the periphery thereof an inclined plane (40) via which said secondary body holds the elastic arm (38) in the housing (36) in the storage position of the back injection device (1).

16. Implant back injection device according to any of claims 11 to 15 in that they depend upon any of claims 9 or 10, **characterized in that** the sheath (6) cooperates with the retaining element (8) to free the passage for the hollow needle (28).

17. Implant back injection device according to claim 16, **characterized in that** the sheath (6) has, on the inner periphery of the distal end thereof at least one inclined plane (32), which abuts on the elastic tongue (24) when the back injection device (1) is pressed against the subject's skin (22), such that said tongue (24) moves away from the rest position thereof and frees the passage for the needle (28), or a snug (48), which, in the storage position of the back injection device (1), bends the elastic tongue (46) towards the inside of the volume of the retaining element (8), said snug (48) moving away from said elastic tongue (46), which returns to the rest position thereof and allows the needle (28) to align with the general axis of forward movement thereof when said back injection device (1) is pressed against the subject's skin (22).

18. Implant back injection device according to claim 17, **characterized in that** the sheath (6) includes two inclined planes (32) between which a rib (34) slides, provided on the retaining element (8) for indexing the position of said retaining element (8) inside said sheath (6).

19. Implant back injection device according to claim 17, **characterized in that** the retaining element (8) includes a groove into which the snug (48) slides for indexing the position of said retaining element (8) relative to the sheath (6).

20. Implant back injection device according to any of claims 11 to 19 in that they depend upon any of claims 6 or 7, **characterized in that** the position of the sheath (6) relative to the main body (10) is indexed by at least one snug (52), which is engaged in the longest groove (54) of the cam path (56).

21. Implant back injection device according to any of claims 5 to 20, **characterized in that** the base (58) includes a tube portion (72), which defines a through aperture (74) for holding the hollow needle (28) and which is connected to the hollow cylindrical body (53) of said base (58) by at least one rib (76).

22. Implant back injection device according to claim 21, **characterized in that** the base (58) is capped by a cap (66) inside which there extends a sleeve (78), a bar (84) extending diametrically inside said sleeve (78).

23. Implant back injection device according to claim 22, **characterized in that** the secondary body (12) is a hollow body of substantially cylindrical shape provided with two diametrically opposite rectilinear slots (102) which extend from the proximal end of said secondary body (12) to a height (h) above the distal end (4) of the back injection device (1), said slots (102) embodying two tube portions (12a, 12b), which pass right through the tube portion (72), penetrating the interior of the hollow cylindrical body (59) of the base (58), then passing right through the bar (58) prior to penetrating the interior of the sleeve (78).

24. Implant back injection device according to claim 23, **characterized in that** the two tube portions (12a, 12b) include locking means (104).

25. Implant back injection device according to claim 24, **characterized in that** the piston rod (88) includes a head (86) via which said piston rod is coupled to the bar (84) to be driven by the main body (10) when the needle (28) penetrates the subject's skin (22), and via which said piston rod cooperates with the locking means (104) in order to be uncoupled from said main body (10) and coupled to the secondary body (12) when the needle (28) is withdrawn from the subject's skin (22).

26. Implant back injection device according to any of claims 5 to 20, **characterized in that** the base (122) includes a tube portion (124), which defines a through hole (126) for holding the hollow needle (28) and which is extended by a rectilinear groove (128) on the opposite flanks (128a) to which a cylinder portion (132) is attached so as to delimit an annular passage (134), an aperture (136) being arranged in the bottom (128b) of said groove (128), the base (122) further including at the proximal end thereof a button (138) in which a notch (146) is made.

27. Implant back injection device according to claim 26, **characterized in that** the secondary body (114) includes a single tube portion (114a), which is capable of sliding into the annular space (134), an elastic arm (118) ending at the free end thereof in a bulge (120) extending into an aperture (116) arranged in the tube portion (114a).

28. Implant back injection device according to claim 27, **characterized in that** the piston rod (88) includes a head (148) via which it is coupled to the button (138) to be driven by the main body (10) when the needle (28) penetrates the subject's skin (22), and via which said piston rod cooperates with the secondary body (114) to remain immobile when the needle (28) is withdrawn from the subject's skin (22).

29. Implant back injection device according to claim 28, **characterized in that** the head (148) of the piston rod (88) includes a snug (150), which, in the storage position of the back injection device (1), projects into the notch (146) of the button (138), the snug (150) being connected via an inclined plane (152) to a heel (154) via which said snug (150) slides over the bulge (120) of the elastic arm (118) and finishes by passing over the latter to fall into the aperture (116), at the moment when the needle (28) is completely pushed into the subject's skin (22), the snug (150) being subsequently retained by the bulge (120), which allows said snug to be uncoupled from said main body (144) gradually as the secondary body (114) exits said main body (144) again, until the moment when said snug (150) is facing the aperture (136) and penetrates the latter, the heel (154) thereof being uncoupled from the bulge (120), such that the piston rod (88) is again coupled to the main body (144), which allows the secondary body (114) to travel the distance necessary for the complete withdrawal of the needle (28) from the subject's skin (22).
